(19) Europäisches Patentamt
European Patent Office
Office européen des brevets

(11) EP 1 447 096 A1

(12) **EUROPEAN PATENT APPLICATION**
published in accordance with Art. 158(3) EPC

(43) Date of publication:
**18.08.2004 Bulletin 2004/34**

(21) Application number: **02785939.6**

(22) Date of filing: **18.11.2002**

(51) Int Cl.⁷: **A61K 45/00**, A61K 31/19, A61K 31/501, A61K 31/553, A61P 13/00, A61P 13/10, A61P 43/00

(86) International application number:
**PCT/JP2002/012000**

(87) International publication number:
**WO 2003/043655 (30.05.2003 Gazette 2003/22)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **19.11.2001 JP 2001353303**

(71) Applicant: **ONO PHARMACEUTICAL CO., LTD.**
**Osaka-shi, Osaka 541-8526 (JP)**

(72) Inventors:
- **MARUYAMA, Takayuki,**
  **c/o Ono Pharmaceutical Co. Ltd**
  **Mishima-gun, Osaka 618-8585 (JP)**
- **NONAKA, Shigeyuki,**
  **c/o Ono Pharmaceutical Co. Ltd.**
  **Mishima-gun, Osaka 618-8585 (JP)**
- **YAMAMOTO, Hiroshi,**
  **c/o Ono Pharmaceutical Co. Ltd.**
  **Osaka-shi, Osaka 541-8526 (JP)**
- **KOBAYASHI, Kaoru,**
  **c/o Ono Pharmaceutical Co. Ltd.**
  **Mishima-gun, Osaka 618-8585 (JP)**

(74) Representative: **Henkel, Feiler & Hänzel**
**Möhlstrasse 37**
**81675 München (DE)**

(54) **REMEDIES FOR URINARY FREQUENCY**

(57) The present invention relates to an agent for the treatment and/or prevention of pollakiuria comprising a compound having an antagonism to an $EP_1$ receptor which is a prostaglandin $E_2$ receptor subtype. A compound having an antagonism to an $EP_1$ receptor antagonistically acts on an $EP_1$ receptor which is a prostaglandin $PGE_2$ receptor subtype and significantly shows a suppressive activity for urination frequency in models where pollakiuria is induced. Therefore, it is effective for the treatment and/or prevention of pollakiuria (that which is due to nurogenic bladder, nervous bladder, stimulated bladder, unstable bladder, benign prostatic hypertrophy, *etc.*).



Printed by Jouve, 75001 PARIS (FR)

## Description

Technical Field

The present invention relates to an agent for the treatment of pollakiuria. More particularly, it relates to an agent for the treatment and/or prevention of pollakiuria comprising a compound having an antagonism to an $EP_1$ receptor which is a prostaglandin $E_2$ receptor subtype.

Background Art

Prostaglandin $E_2$ (hereinafter, abbreviated as $PGE_2$) has been known as a metabolite in an arachidonic acid cascade and has been known to have cytoprotective activity, uterine contractile activity, a pain-inducing effect, a promoting effect on digestive peristalsis, an awakening effect, a suppressive effect on gastric acid secretion, hypotensive activity and diuretic activity, *etc*.
During the studies in recent years, it has been clarified that, in $PGE_2$ receptor, there are subtypes having each different role. When roughly classified, there are four subtypes which have been known up to now and each of them is called $EP_1$, $EP_2$, $EP_3$ and $EP_4$ (*J. Lipid Mediators Cell Signaling,* 12, 379-391 (1995)).
Since $PGE_2$ has so many physiological activities, it has a disadvantage that an effect which is other than the objective effect becomes a side effect and studies have been continued for overcoming the disadvantage by investigating the role of each subtype so as to prepare a compound which is useful only for the subtype.
Among those subtypes, an $EP_1$ receptor has been known to be related to a pain-inducing effect, a fever-inducing effect and diuresis (q.v. *Br. J. Pharmacol.,* 1994, 112, 735-40; *European J. Pharmacol.,* 152 (1988) 273-9; and *Gen Pharmacol.,* Sep 1992, 23(5), 805-9). Therefore, a compound which antagonizes the receptor is supposed to be effective as analgesic, antipyretic and an agent for the treatment of pollakiuria.
However, at present, it has not been specifically clarified yet whether a compound having an antagonism to an $EP_1$ receptor is really has a suppressive activity for pollakiuria.
On the other hand, urinary disturbance is classified into urine storage disorder where urine is unable to be retained upon storage of urine and voiding disfunction where it is not possible to excrete urine with a sufficient excreting force. Main symptoms for the urine storage disorder are pollakiuria (which is due to nurogenic bladder, nervous bladder, stimulated bladder, unstable bladder, benign proststatic hypertrophy, *etc*.), urgency and urinary incontinence. As to the causes for those disturbances, there are abnormality in a urine storage function of bladder which is caused by hypertonic detrusor and resistance of an exit area of bladder which is caused by decrease in sphincter muscle function of the urethra. Between them, disturbance caused by hypertronic detrusor are those that caused by abnormal contraction of detrusor and that caused by hyperesthesia of bladder.
For the urine storage disorder caused by abnormal detrusor, agents which decrease the contraction of detrusor mainly comprising an anticholinergic agent have been used. However, the agents such as an anticholinergic agent which cause decrease of the contraction of detrusor also suppress contraction upon urination when contraction of detrusor is necessary. Therefore, an increase in residual urine is a problem. Dry mouth caused by an anticholinergic effect is also pointed out as a main side effect of such agents.
With regard to an $EP_1$ antagonist, the followings have been known for example.
In WO 98/27053, compounds represented by formula (A)

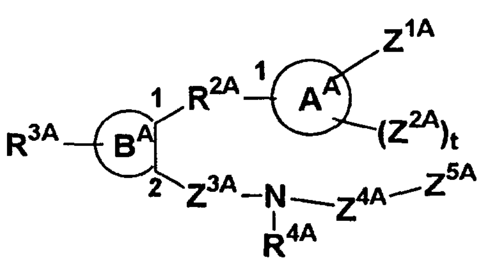

(A)

(in the formula, all groups have the same meanings which will be defined later) have been mentioned to be useful as analgesic, and for the treatment and/or the prevention of pollakiuria. However, in the specification, only examples for a binding experiment to an $EP_1$ receptor are mentioned and there is no description for the relation to specific diseases.
In EP 878465, compounds represented by formula (B)

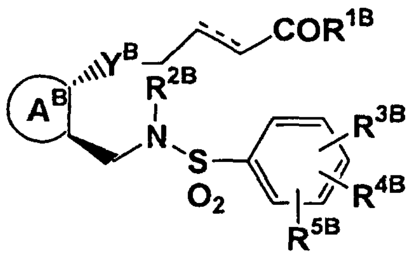

(B)

(in the formula, all groups have the same meanings which will be defined later) have been mentioned to be useful as analgesics and antipyretic and for the treatment and/or the prevention of pollakiuria. In the specification, a relationship between $EP_1$ receptor antagonism and pollakiuria is suggested but there no specific experiment and evidence therefor.

In WO 92/19617 (JP-A-6-507408), compounds represented by formula (C)

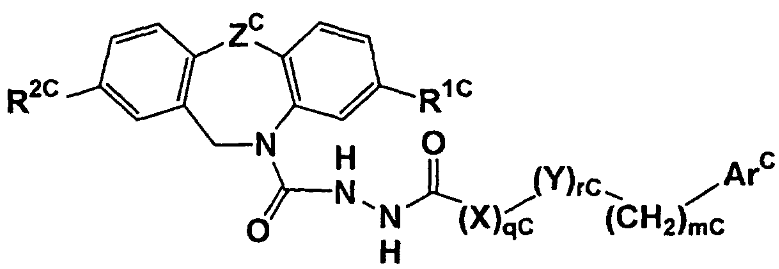

(C)

(in the formula, all groups have the same meanings which will be defined later) have been mentioned to be useful for the treatment of central nervous system disturbance such as pain, convulsion and ischemia as well as osteoporosis, dysmenorrhea, asthma, enuresis, arrhythmia and diarrhea. In its specification, PGE and analgesic activity are measured but there is no specific experiment and evidence for pollakiuria.

Compounds represented by formula (D)

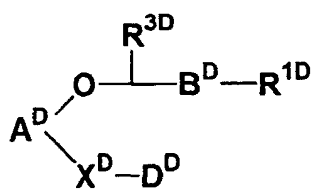

(D)

(in the formula, all groups have the same meanings which will be defined later) mentioned in WO 96/06822 (JP-A-10-504836) and compounds represented by formula (E)

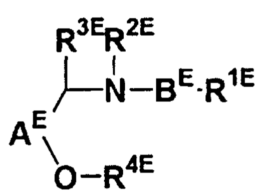

(E)

(in the formula, all groups have the same meanings which will be defined later) mentioned in WO 97/00863 are mentioned to be useful for the treatment of pain [such as pain accompanied by symptom of joint (such as articular rheumatism and osteoarthritis), pain accompanied by postoperative pain, puerperal pain and dental symptom (such as dental caries and osteogenic inflammation) and pain accompanied by burn (including sunburn)] and osteopathia (such as osteoporosis, malignant hypercalcemia and Behçet's disease) and as a measure for pain accompanied by external

injury as a result of physical exercise and sprain, and all pain states where prostaglandin of type E plays a pathological physiology as whole or partially. In those specifications, there are description concerning an antagonism to $EP_1$ receptor, but there is no description of the relation between $EP_1$ receptor and pollakiuria.

Compounds represented by formula (F)

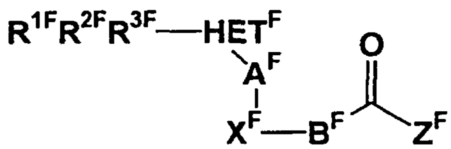

(F)

(in the formula, all groups have the same meanings which will be defined later) mentioned in WO 99/47497 and compounds represented by formula (G)

$$Ar^{1G}\text{-}W^{G}\text{-}Ar\text{-}^{G}\text{-}X^{G}\text{-}W^{G} \qquad (G)$$

(in the formula, all groups have the same meanings which will be defined later) mentioned in WO 00/20371 are mentioned to be used for the treatment or the prevention of diseases caused by prostaglandin and are suggestions are mentioned to pain, fever or inflammation by rheumatic fever, influenza or other viral infection, common cold, pain of the back and neck, skeletal pain, postpartum pain, dysmenorrhea, headache, migraine, tooth pain, sprain or fracture, myositis, neuralgia, synovitis, arthritis, rheumatic arthritis, degenerative joint diseases (osteoarthritis), gout or ankylosing spondylitis, bursitis, burn including radioactive and corrosive chemical injury, sunburn, pain after surgical operation or dental treatment, immune diseases or autoimmune diseases, cytoneoplastic degeneration or development of metastatic tumor, diabetic retinopathy, tumoral angiogenesis, prostanoids-inducing smooth muscle contraction related to dysmenorrhea, early delivery, diseases related to asthma and eosinophil leukocyte, Alzheimer's disease, glaucoma, osteopenia, osteoporosis, osteogenetic acceleration, Behçet's disease, protection of cell in pepsin-like tumor, gastritis, topical enteritis, ulcerative colitis, diverticulitis, other gastrointestinal hindrances, gastrointestinal bleeding, patients during chemotherapy, hypoprothrombinemia, hemophilia and other coagulation diseases such as bleeding problem, renal diseases, thrombosis, obstructive blood vessel disturbance and anticoagulation.

Other known compounds acting a $PGE_2$ receptor, particularly $EP_1$ antagonists, are 2,3,6-substituted-4-pyrone derivatives mentioned in the specification of US 4132847, N-alkenyl-3-hydrobenzo[b]thiophene-2-carboxyamide derivatives mentioned in the specification of EP 160408, dibenzoxepinecarboxylic acid hydrazide derivatives mentioned in the specification of EP 193822, 8-chlorodibenzoxazepine-10-carboxylic acid hydrazide derivatives mentioned in the specification of EP 218077, cyclohept[b]indolealkane derivatives mentioned in the specification of US 4775680, 9-benzyldifluorotetrahydrocarbazolylacetic acid derivatives mentioned in the specification of EP 300676, tricyclic hetero ring derivatives mentioned in the specification of EP 480641, dibenzoxazepine derivatives mentioned in the specification of EP 512399, dibenzoxazepine derivatives mentioned in the specification of EP 512400, tricyclic hetero ring derivatives mentioned in the specification of EP 534667, 10-acyldibenzoxazepine, thiazepine or diazeptine derivative mentioned in the specification of WO 93/07132, dibenzoxazepine derivative mentioned in the specification of EP 539977, dibenzoxazepine or dibenzothiazepine derivatives mentioned in the specification of WO 93/13082, dibenzoxazepinecarboxylic acid derivatives mentioned in the specification of US 5281590, N-carbazylbenzoxazepine derivative mentioned in the specification of US 530464, dibenzoxazepine derivatives mentioned in the specification of US 5324722, dibenzthia- or oxazepinyl-3-cyclobutene-1,2-dione derivatives mentioned in the specification of US 5354746, dibenzoxazepine or dibenzothiazepine derivatives mentioned in the specification of US 5354747, dibenzoxazepine or dibenzothiazepine derivatives mentioned in the specification of US 5420270, azepine derivatives mentioned in the specification of US 5441950, dibenzoxazepine derivatives mentioned in the specification of EP 694546, o-arylmethoxy-arylmethylamino aromatic acid derivatives mentioned in the specification of WO 96/03380, dibenzoxazpine or dibenzothiazepine derivative mentioned in the specification of US 5504077, aromatic derivatives mentioned in the specification of WO 96/11902, o-substituted aromatic compounds mentioned in the specification of EP 752421 and aromatic derivatives mentioned in the specification of WO 97/00864.

However, in those specifications, there is no description of relationship between the subtype of $PGE_2$ receptor and the specific diseases. There is no description of relationship between the $EP_1$ antagonism and pollakiuria.

As mentioned above, in $PGE_2$ receptor, there are four subtypes ($EP_1$, $EP_2$, $EP_3$ and $EP_4$) having different role and each of them participates in different pharmacological activity. Although there has been known that, among them, compounds having an antagonism to $EP_1$ receptor are effective for pollakiuria, it has not been specifically known that compounds having an antagonism to $EP_1$ receptor has a suppressive action to pollakiuria.

Disclosure of the Invention

The inventors of the present invention have carried out intensive investigations for finding compounds which are effective for the treatment and/or the prevention of pollakiuria, and found that the compounds having an antagonism to an $EP_1$ receptor was actually applied to pollakiuria-inducing model of animals to suppress the pollakiuria and achieved the present invention. In the previous documents mentioned in the Background Art, no relation was shown between subtypes of $PGE_2$ receptors and the specific diseases thereby and no relation between $EP_1$ antagonism and pollakiuria was suggested as well. Accordingly, it is not possible to anticipate from the description of those documents that the compound having an $EP_1$ antagonism is effective to pollakiuria (the present invention).

Thus, the present invention relates to agents for the treatment and/or prevention comprising a compound having an antagonism to an $EP_1$ receptor which is a prostaglandin $E_2$ receptor subtype for pollakiuria (that which is due to nurogenic bladder, nervous bladder, stimulated bladder, unstable bladder, benign proststatic hypertrophy, *etc*.).

Compounds having an antagonism to an $EP_1$ receptor used in the present invention include not only $EP_1$ receptor antagonists which have been known but also any $EP_1$ receptor antagonists which will be found in future. The following compounds are preferably used (Details of the definitions of symbols in the formulae shown hereinafter are the same as those mentioned in each of the specifications).

(1) A sulfonamide or carboamide derivative represented by formula (A) mentioned in the specification of WO 98/27053

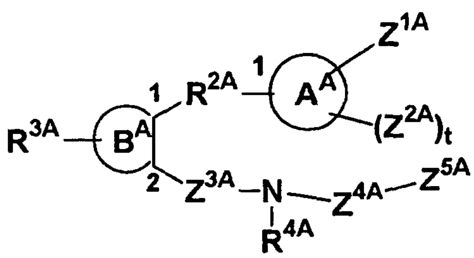

(A)

(in the formula,

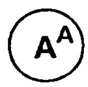 and 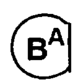

each independently is a $C_{5-15}$ carbon ring or a five- to seven-membered hetero ring having 1 or 2 oxygen, sulfur or nitrogen atom(s),

$Z^{1A}$ is

-$COR^{1A}$,
-$C_{1-4}$ alkylene-$COR^{1A}$,
-CH=CH-$COR^{1A}$,
-C≡C-$COR^{1A}$ or
-O-$C_{1-3}$ alkylene-$COR^{1A}$

(in each formula, $R^{1A}$ is a hydroxyl group, $C_{1-4}$ alkoxy or a group represented by a formula $NR^{6A}R^{7A}$ (in the formula, $R^{6A}$ and $R^{7A}$ each independently is a hydrogen atom or $C_{1-4}$ alkyl) or -$C_{1-5}$ alkylene-OH,

$Z^{2A}$ is a hydrogen atom, $C_{1-4}$ alkyl, $C_{1-4}$ alkoxy, nitro, halogen, trifluoromethyl, trifluoromethoxy, a hydroxyl group or a group represented by the formula $COR^{1A}$ (in the formula, $R^{1A}$ has the same meaning as defined above),

$Z^{3A}$ is a single bond or $C_{1-4}$ alkylene,

$Z^{4A}$ is $SO_2$ or CO,

$Z^{5A}$ is

(1) $C_{1-8}$ alkyl, $C_{2-8}$ alkenyl, or $C_{2-8}$ alkynyl,

(2) phenyl, $C_{3-7}$ cycloalkyl, a five- to seven-membered hetero ring having one or two oxygen, sulfur or nitrogen atom(s),

(3) phenyl or $C_{3-7}$ cycloalkyl-substituted $C_{1-4}$ alkyl, $C_{2-4}$ alkenyl or $C_{2-4}$ alkynyl,

(in the above (2) and (3), phenyl, $C_{3-7}$ cycloalkyl and a five- to seven-membered hetero ring having one or two oxygen, sulfur or nitrogen atom(s) may be substituted with one to five $R^{5A}$ group(s) (each of plural $R^{5A}$ independently is a hydrogen atom, $C_{1-6}$ alkyl, $C_{1-6}$ alkoxy, $C_{1-6}$ alkylthio, nitro, halogen, trifluoromethyl, trifluoromethoxy or a hydroxyl group)),

$R^{2A}$ is

$CONR^{8A}$,
$NR^{8A}CO$,
$CONR^{8A}$-$C_{1-4}$ alkylene,
$C_{1-4}$ alkylene-$CONR^{8A}$,
$NR^{8A}CO$-$C_{1-4}$ alkylene,
$C_{1-4}$ alkylene-$NR^{8A}CO$,
$C_{1-3}$ alkylene-$CONR^{8A}$-$C_{1-3}$ alkylene,
$C_{1-3}$ alkylene-$NR^{8A}CO$-$C_{1-3}$ alkylene

(in each formula, $R^{8A}$ is a hydrogen atom or $C_{1-4}$ alkyl),

O, S, $NZ^{6A}$

(in the formula, $Z^{6A}$ is a hydrogen atom or $C_{1-4}$ alkyl),

$Z^{7A}$-$C_{1-4}$ alkylene,
$C_{1-4}$ alkylene-$Z^{7A}$,
$C_{1-3}$ alkylene-$Z^{7A}$-$C_{1-3}$ alkylene

(in the formulae, $Z^{7A}$ is O, S or a group represented by the formula $NZ^{6A}$ (in the formula, $Z^{6A}$ has the same meaning as defined above)),

CO,
CO-$C_{1-4}$ alkylene,
$C_{1-4}$ alkylene-CO,
$C_{1-3}$ alkylene-CO-$C_{1-3}$ alkylene,
$C_{2-4}$ alkylene,
$C_{2-4}$ alkenylene or
$C_{2-4}$ alkynylene

$R^{3A}$ is a hydrogen atom, $C_{1-6}$ alkyl, $C_{1-6}$ alkoxy, $C_{1-6}$ alkylthio, nitro, halogen, trifluoromethyl, trifluoromethoxy, hydroxyl group or hydroxymethyl,

$R^{4A}$ is

(1) a hydrogen atom,
(2) $C_{1-8}$ alkyl, $C_{2-8}$ alkenyl or $C_{2-8}$ alkynyl,
(3) $C_{1-6}$ alkyl substituted with one or two group(s) selected from the group consisting of $COOZ^{8A}$, $COZ^{9A}Z^{10A}$, $OZ^{8A}$ group (in each group, $Z^{8A}$, $Z^{9A}$ and $Z^{10A}$ each independently is a hydrogen atom or $C_{1-4}$ alkyl) and $C_{1-4}$ alkoxy-$C_{1-4}$ alkoxy,
(4) $C_{3-7}$ cycloalkyl,
(5) phenyl or $C_{3-7}$ cycloalkyl-substituted $C_{1-4}$ alkyl, $C_{2-4}$ alkenyl or $C_{2-4}$ alkynyl,

(phenyl and $C_{3-7}$ cycloalkyl in the above (4) and (5) may be substituted with one to five $R^{5A}$ group(s) ($R^{5A}$ has the same meaning as defined above)) and

$n^A$ and $t^A$ each independently is an integer of 1 to 4,
wherein

(1) $R^{2A}$ and $Z^{3A}$ each binds to only 1- and 2-position of

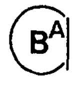

and
(2) when

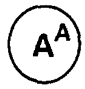

is a benzene ring and $(Z^{2A})_t{}^A$ is not $COR^{1A}$, then $Z^{1A}$ binds to only 3- or 4-position of the benzene ring.), or a non-toxic salt thereof.

(2) A benzenesulfonamide derivative represented by formula (B) mentioned in the specification of EP 878465

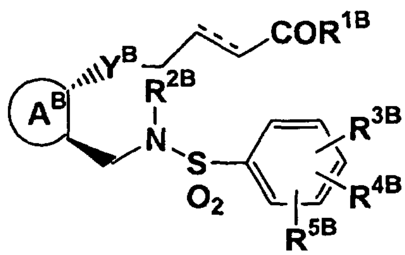

(B)

(in the formula,

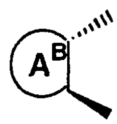

is a group represented by

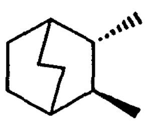
(a)

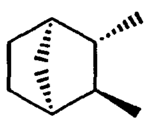
(b)

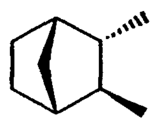
(c)

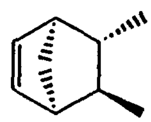
(d)

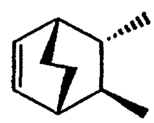
(e)

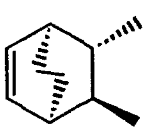
(f)

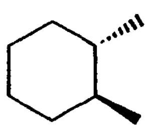
(g)

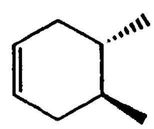
(h)

or

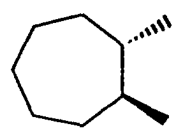
(i)

$R^{1B}$ is hydroxy, a $C_{1-4}$ alkoxy group or a group represented by formula $NR^{6B}N^{7B}$ (in the formula, $R^{6B}$ and $R^{7B}$ each independently is a hydrogen atom or a $C_{1-4}$ alkyl group),
**[0024]** $R^{2B}$ is a hydrogen atom or a $C_{1-4}$ alkyl group,
**[0025]** $R^{3B}$ and $R^{4B}$ each is a $C_{1-4}$ alkyl group, a halogen atom or trifluoromethyl group,
**[0026]** $R^{5B}$ is a hydrogen atom, a $C_{1-4}$ alkyl group, a halogen atom or trifluoromethyl group,
**[0027]** $Y^B$ is cis-vinylene or trans-vinylene and
a symbol 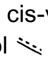 is a single bond or a double bond,
whereinwhen

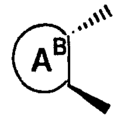

is a formula

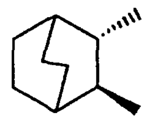

$R^{1B}$ is hydroxy or a $C_{1-4}$ alkoxy group, $R^{2B}$ is a hydrogen atom, $Y^B$ is cis-vinylene and the symbol 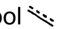 is a single bond, then

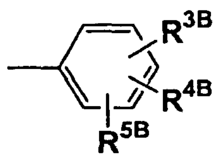 is not 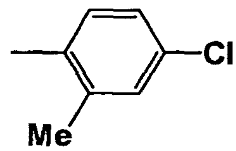 .),

or a non-toxic salt thereof or a cyclodextrin clathrate thereof.

(3) A compound represented by formula (C) mentioned in the specification of WO 92/19617

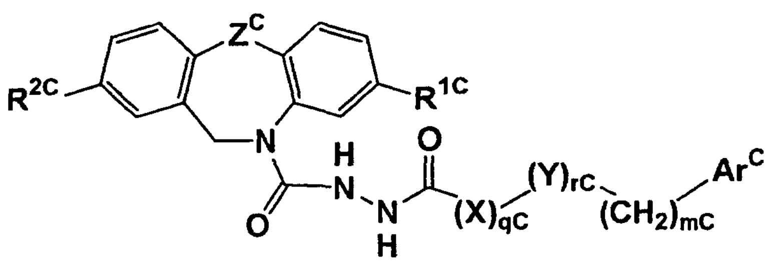

(C)

(in the formula, $R^{1C}$ is a hydrogen atom, halogen or $-CF_3$;
$R^{2C}$ is a hydrogen atom, halogen, -OH or $-OCH_3$;
$Z^C$ is -O-, -S-, -S(O)- or $-S(O)_2-$;
$X^C$ is -CH=CH-, $-CF_2-$, -CHF-, $-(CH_2)_{nC}-$ or $-(CH_2)_{pC}$-CH=CH-;
$Y^C$ is -CH(OH)-, $-NR^{3C}-$, -S-, -S(O)-, $-S(O)_2-$ or -O-;
$q^C$ is 0 or 1;
$r^C$ is 0 or 1 (wherein when

(1) $X^C$ is -CH=CH-, $-(CH_2)_{nC}-$ or $-(CH_2)_{pC}$-CH=CH-, $q^C$ is 1 and $Ar^C$ is imidazole or phenyl,
(2) X is $-(CH_2)_{nC}$, $q^C$ is 1, $n^C$ is 1 and $Ar^C$ is halogen, methyl or alkoxy-substituted ethylphenyl or
(3) $q^C$ is 1, $m^C$ is 1, 2, 3, 4, 5 or 6 and $Ar^C$ is imidazole or phenyl, then $r^C$ is not 0);
$m^C$ is 0 to 6 (wherein when xC is $-(CH_2)_{nC}-$, $q^C$ is 1, $Y^C$ is -O-, -S-, -S(O)- or $-S(O)_2-$ and $Ar^C$ is phenyl, then $m^C$ is not 0);
$n^C$ is an integer of 1 to 6;
$p^C$ is an integer of 1 to 6;
$R^{3C}$ is a hydrogen atom or tert-butyloxycarbonyl and
$Ar^C$ is aryl, alkyl-substituted aryl or aryl-substituted aryl).
(4) A compound represented by formula (D) mentioned in the specification of WO 96/06822

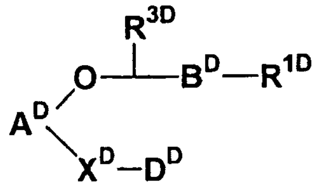

(D)

(except for 4-(2-benzyl-3-hydroxy-4-formylphenoxymethyl)-3- methoxybenzoic acid and 4-(2-(3-phenylprop-2-en-1-yl)-3- hydroxy-4-formylphenoxymethyl-3-methoxybenzoic acid)

(in the formula, $A^D$ is optionally-substituted eight- to ten-membered bicyclic heteroaryl, five- or six-membered heteroaryl, naphthyl or phenyl where the binding groups $-OCH(R^{3D})-$ and $-X^D-$ are positioned at 1- and 2-positions each other on a cyclic carbon atoms,

$B^D$ is an optionally-substituted five- or six-membered heteroaryl ring or optionally-substituted phenyl,

$D^D$ is optionally substituted pyridyl, pyrazinyl, pyrimidyl, pyridazinyl, pyrrolyl, thienyl, furyl, pyrazolyl, thiazolyl, isothiazolyl, oxazolyl, isoxazolyl or phenyl,

$X^D$ is a formula $-(CHR^{4D})_{nD}-$ or $-(CHR^{4D})_{pD}CR^{4D}=CR^{4D}(CHR^{4D})_{qD}-$ in which $n^D$ is 1 to 3 and both $p^D$ and $q^D$ are 0 or one of $p^D$ and $q^D$ is 1 while another is 0,

$R^{1D}$ is positioned on the ring $B^D$ in a relation of 1,3 or 1,4 with a binding group $-OCH(R^{3D})-$ on a six-membered ring or in a relation of 1,3-with a binding group $-OCH(R^{3D})-$ on a five-membered ring and is carboxy, carboxy-$C_{1-3}$ alkyl, tetreazolyl, tetrazolyl-$C_{1-3}$ alkyl, tetronic acid, hydroxamic acid or sulfonic acid, or $R^{1D}$ is a formula $-CONR^{aD}R^{a1D}$ in which $R^{aD}$ is a hydrogen atom or $C_{1-6}$ alkyl, $R^{a1}$ is a hydrogen atom or optionally substituted $C_{1-6}$ alkyl, $C_{2-6}$ alkenyl, $C_{2-6}$ alkynyl, $C_{3-7}$ cycloalkyl, $C_{3-7}$ cycloalkyl-$C_{1-6}$ alkyl, $C_{3-7}$ cycloalkyl-$C_{2-6}$ alkenyl, $C_{3-7}$ cycloalkyl-$C_{2-6}$ alkynyl, $C_{5-7}$ cycloalkenyl, $C_{3-7}$ cycloalkenyl-$C_{1-6}$ alkyl, $C_{5-7}$ cycloalkenyl-$C_{2-6}$ alkenyl, $C_{5-7}$ cycloalkenyl-$C_{2-6}$ alkynyl, $C_{1-3}$ alkyl which is substituted with a five- or six-membered saturated or a partially saturated hetero ring, five- or six-membered saturated or a partially saturated hetero ring or five- or six-membered heteroaryl or, in the formula, $R^{aD}$ and $R^{a1D}$ form an amino acid residue or ester thereof together with an amide nitrogen ($NR^{aD}R^{a1D}$) to which they bind, or $R^{1D}$ is a formula $-CONHSO_2R^{bD}$ in which $R^{bD}$ is optionally substituted $C_{1-6}$ alkyl, $C_{2-6}$ alkenyl, $C_{2-6}$ alkynyl, $C_{3-7}$ cycloalkyl-$C_{1-6}$ alkyl, $C_{3-7}$ cycloalkyl-$C_{2-6}$ alkenyl, $C_{3-7}$ cycloalkyl-$C_{2-6}$ alkynyl, $C_{3-7}$ cycloalkenyl-$C_{1-6}$ alkyl, $C_{3-7}$ cycloalkenyl-$C_{2-6}$ alkenyl, $C_{3-7}$ cycloalkenyl-$C_{2-6}$ alkynyl, five- or six-membered heteroaryl, five- or six-membered heteroaryl-$C_{1-6}$ alkyl, phenyl, phenyl-$C_{1-6}$ alkyl, five- or six-membered saturated or a partially saturated hetero ring or five- or six-membered saturated or a partially saturated hetero ring-$C_{1-6}$ alkyl,

$R^{3D}$ is a hydrogen atom or $C_{1-4}$ alkyl and

$R^{4D}$ is a hydrogen atom or $C_{1-4}$ alkyl), or an N-oxide thereof in case chemically possible, a sulfur oxide having ring in case chemically possible, a pharmaceutically acceptable salt thereof, or an ester or amide hydrolyzable in the living body.

(5) A compound of formula (E) mentioned in WO 97/00863

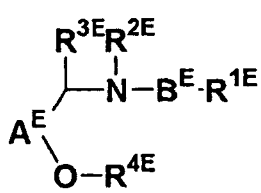

(E)

(in the formula $A^E$ is the following which is optionally substituted:

phenyl, naphthyl, pyridyl, pyrazinyl, pyridazinyl, pyrimidyl, thienyl, thiazolyl, oxazolyl or thiadiazolyl having at least two adjacent ring carbon atoms;

in that case, $-CH(R^{3E})N(R^{2E})B^E-R^{1E}$ and $-OR^{4E}$ are positioned at 1 and 2 each other on the ring carbon atoms and the ring atom positioned at ortho to an $OR^{4E}$ binding group (and, therefore, at 3-position on the basis of a-$CHR^{3E}NR^{2E}$- binding group) is not substituted;

$B^E$ is the following which is optionally substituted:

phenyl, pyridyl, thiazolyl, oxazolyl, thienyl, thiadiazolyl, imidazolyl, pyrazinyl, pyridazinyl or pyrimidyl;

$R^{1E}$ is positioned 1,3 or 1,4 to a -$CH(R^{3E})N(R^{2E})$- binding group on a ring $B^E$ and $R^{1E}$ is carboxy, carboxy-$C_{1-3}$ alkyl, tetrazolyl, tetrazoly-$C_{1-3}$ alkyl, tetronic acid, hydroxamic acid or sulfonic acid or $R^{1E}$ is -$CONR^{aE}R^{a1E}$ [in that case, $R^{aE}$ is a hydrogen atom or $C_{1-6}$ alkyl and $R^{a1E}$ is a hydrogen atom, $C_{1-6}$ alkyl (in some cases, it is substituted with halogen, amino, $C_{1-4}$ alkylamino, di-$C_{1-4}$ alkylamino, hydroxy, nitro, cyano, trifluoromethyl, $C_{1-4}$ alkoxy or $C_{1-4}$ alkoxycarbonyl), $C_{2-6}$ alkenyl (in that case, a double bond is not at 1-position), $C_{2-6}$ alkynyl (in that case, a triple bond is not at 1-position), carboxyphenyl, five- or six-membered heterocyclic $C_{1-3}$ alkyl, five- or six-membered heteroaryl $C_{1-3}$ alkyl, five- or six-membered heterocyclic or five- to six-membered heteroaryl or $R^{aE}$ and $R^{a1E}$ form an amino acid residue or ester thereof together with an amide nitrogen ($NR^{aE}R^{a1E}$) to which they bind] or $R^1$ is a group of formula -$CONHSO_2R^b$ [in that case, $R^{bE}$ is $C_{1-6}$ alkyl (in some cases, it may be substituted with halogen, hydroxy, nitro, cyano, trifluoromethyl, $C_{1-4}$ alkoxy, amino, $C_{1-4}$ alkylamino, di-$C_{1-4}$ alkylamino or $C_{1-4}$ alkoxycarbonyl), $C_{2-6}$ alkenyl (in that case, a double bond is not at 1-position), $C_{2-6}$ alkynyl (in that case, a triple bond is not at 1-position), five- or six-membered heterocyclic $C_{1-3}$ alkyl, five- or six-membered heteroaryl $C_{1-3}$ alkyl, five- or six-membered heterocyclic, five- or six-membered heteroaryl or phenyl];

in that case, any heterocyclic or heteroaryl group in $R^{a1E}$ is optionally substituted with halogen, hydroxy, nitro, hydroxy, amino, cyano, $C_{1-6}$ alkoxy, $C_{1-6}$ alkyl $S(O)_p{}^{E}$- ($p^E$ is 0, 1 or 2), $C_{1-6}$ alkylcarbamoyl, $C_{1-4}$ alkylcarbamoyl, di-($C_{1-4}$ alkyl)carbamoyl, $C_{2-6}$ alkenyl, $C_{2-6}$ alkynyl, $C_{1-4}$ alkoxycarbonylamino, $C_{1-4}$ alkanoylamino, $C_{1-4}$ alkanoyl(N-$C_{1-4}$ alkyl)amino, $C_{1-4}$ alkanesulfonamido, benzenesulfonamido, aminosulfonyl, $C_{1-4}$ alkylaminosulfonyl, di($C_{1-4}$ alkyl)aminosulfonyl, $C_{1-4}$ alkoxycarbonyl, $C_{1-4}$ alkanoyloxy, $C_{1-6}$ alkanoyl, formyl $C_{1-4}$ alkyl, hydroxyimino $C_{1-6}$ alkyl, $C_{1-4}$ alkoxyimino $C_{1-6}$ alkyl or $C_{1-6}$ alkylcarbamoylamino; or

$R^{1E}$ is a group of formula -$SO_2N(R^{cE})R^{c1E}$ [in that case, $R^{cE}$ is hydrogen or $C_{1-4}$ alkyl and $R^{c1E}$ is a hydrogen atom or $C_{1-4}$ alkyl]; or r1 is a group of the following formula ($E^A$), ($E^B$) or ($E^C$):

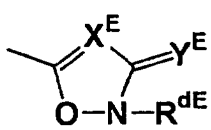
($E^A$)

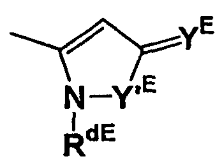
($E^B$)

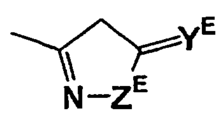
($E^C$)

and, in the above formulae, $X^E$ is CH or a nitrogen atom, $Y^E$ is an oxygen atom or a sulfur atom, $Y'^E$ is an oxygen atom or $NR^{dE}$ and $Z^E$ is $CH_2$, $NR^{dE}$ or an oxygen atom and, in that case, there is one or less ring oxygen and there are at least two ring hetero atoms and, in the above formulae, $R^{dE}$ is a hydrogen atom or $C_{1-4}$ alkyl;

$R^{2E}$ is hydrogen or optionally hydroxy-, cyano- or trifluoromethyl-substituted $C_{1-6}$ alkyl, $C_{2-6}$ alkenyl (in that case, a double bond is not at 1-position), $C_{2-6}$ alkynyl (in that case, a triple bond is not at 1-position), phenyl $C_{1-3}$ alkyl or pyridyl $C_{1-3}$ alkyl;

$R^{3E}$ is a hydrogen atom, methyl or ethyl; and

$R^{4E}$ is an optionally substituted following:

$C_{1-6}$ alkyl, $C_{3-7}$ cycloalkyl $C_{1-3}$ alkyl or $C_{3-7}$ cycloalkyl), or an N-oxide of -$NR^{2E}$- in case chemically possible, or an S-oxide of a sulfur-containing ring in case chemically possible or a pharmaceutically acceptable salt thereof or a hydrolyzable ester or amide in the living body (in that case however, 2-[2-methoxybenzylamino] pyridine-5-carboxylic acid, 4-[2-methoxybenzylamino]benzoic acid, 5-[2,3-dimethoxy- benzylamino]-2-chloro-3-aminosulfonylbenzoic acid and 5-[2,5-dimethoxybenzylamino]-2-hydroxybenzoic acid are to be excluded).

(6) A compound represented by formula (F) mentioned in the specification of WO 99/47497

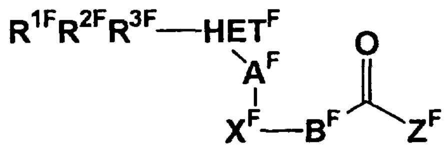

(F)

(in the formula, $HET^F$ is a five- to twelve-membered monocyclic or a bicyclic aromatic ring containing 0 to 3 hetero atom(s) selected from O, $S(O)_{nF}$ and $N(O)_{mF}$ in which $m^F$ is 0 or 1 and $n^F$ is 0, 1 or 2,

$A^F$ is one or two atomic moiety(ies) and is $-W^F-$, -C(O)-, $-C(R^{7F})-W^F-$, $-W-C(R^{7F})_2-$, $-CR^{7F}(OR^{20F})-$, $-C(R^{7F})_2-$, $-C(R^{7F})_2-C(OR^{20F})R^{7F}-$, $-C(R^{7F})_2-C(R^{7F})_2-$ or $-CR^{7F}=CR^{7F}-$ in which $W^F$ is O, $S(O)_{nF}$ or $NR^{17F}$

$X^F$ is five- to ten-membered monocyclic, bicyclic or five- to 10-membered monocyclic or bicyclic heteroaryl having 1 to 3 hetero atom(s) selected from O, $S(O)_{nF}$ and $N(O)_{mF}$ which may be substituted with $R^{14F}$ and $R^{15F}$ where $A^f$ and $B^F$ bind to ortho position of aryl or heteroaryl,

$B^F$ is $-(C(R^{18F})_2)_{pF}-Y^F-(C(R^{18F})_2)_{qF}$ where $p^F$ and $q^F$ each independently is 0 to 3,

$Y^F$ is O, $S(O)_{nF}$, $NR^{17F}$, a single bond or $-CR^{18F}=CR^{18F}-$ and, when $Y^F$ is O, $S(O)_{nF}$, $NR^{17F}$ or $-CR^{18F}=CR^{18F}-$, $p^F + q^F$ is 0 to 6 while, when $Y^F$ is a single bond, $p^F + q^F$ is 1 to 6,

$Z^F$ is OH or $NHSO_2R^{19F}$,

$R^{1F}$, $R^{2F}$ and $R^{3F}$ each independently is H, a halogen atom, lower alkyl, lower alkenyl, lower alkynyl, lower alkenyl-$HET^F(R^{aF})$4-9-, $-(C(CR^{4F})_2)_{pF})SR^{5F}$,$-(C(R^{4F})_2)_{pF})OR^{8F}$, $-(C(R^{4F})_2)_{pF}N(R^{6F})_2$, CN, $NO_2$, $-(C(R^{4F})_2)_{pF}C(R^{7F})_3$, $-COOR^{9F}$, $-CON(R^{6F})_2$ or $-(C(R^{4F})_2)_{pF}SS(O)_{nF}R^{10F}$,

each $R^{4F}$ is H, F, $CF_3$ or lower alkyl, or

two $R^{4F}$ are taken in conjunction and represent a at most six membered ring which may have one heteroatom selected from O, $S(O)_{nF}$ and $N(O)_{mF}$,

each $R^{5F}$ independently is lower alkyl, lower alkenyl, lower alkynyl, $CF_3$, lower alkyl-$HET^F$, lower alkenyl-$HET^F$ or $-(C(R^{18F})_2)_{pF}Ph(R^{11F})_{0-2}$,

each $R^{6F}$ independently is H, lower alkyl, lower alkenyl, lower alkynyl, $CF_3$, phenyl or benzyl, or two $R^{6F}$ binding to N are taken in conjunction and represent a at most six membered ring which may have additional heteroatom selected from O, $S(O)_{nF}$ and $N(O)_{mF}$,

each $R^{7F}$ independently is H, F, $CF_3$ or lower alkyl, or two $F^{7F}$ are taken in conjunction and represent three- to six-membered aromatic or an aliphatic ring containing 0 to 2 heteroatom(s) selected from O, $S(O)_{nF}$ and $N(O)_{mF}$,

each $R^{8F}$ is H or $R^{SF}$,

each $R^{9F}$ independently is H, lower alkyl, lower alkenyl, lower alkynyl, phenyl or benzyl,

each $R^{10F}$ independently is lower alkyl, lower alkenyl, lower alkynyl, $CF_3$, $Ph(R^{11F})_{0-3,}$ $CH_2Ph(R^{11F})_{0-3}$ or $N(R^{6F})_2$,

each $R^{11F}$ independently is lower alkyl, $SR^{20F}$, $OR^{20F}$, $N(R^{6F})_2$, $-COOR^{12F}$, $-CON(R^{6F})_2$, $-COR^{12F}$, CN, $CF_3$, $NO_2$ or a halogen atom,

each $R^{12F}$ independently is H, lower alkyl or benzyl,

each $R^{13F}$ independently is H, a halogen atom, lower alkyl, O-lower alkenyl, S-lower alkyl, $N(R^{6F})_2$, $COOR^{12F}$, CN, $CF_3$ or $NO_2$,

$R^{14F}$ and $R^{15F}$ each independently is lower alkyl, a halogen atom, $CF_3$, $OR^{16F}$, $S(O)_{nF}R^{16F}$ or $C(R^{16F})_2\,OR^{17F}$,

each $R^{16F}$ independently is H, lower alky, lower alkenyl, phenyl, benzyl or $CF_3$,

each $R^{17F}$ independently is H, lower alkyl or benzyl,

each $R^{18F}$ independently is H, F or lower alkyl, or two $R^{18F}$ are taken in conjunction and represent a three- to six-membered ring which may contain one hetero atom selected from O, $S(O)_{nF}$ and N,

each $R^{19F}$ independently is lower alkyl, lower alkenyl, lower alkynyl, $CF_3$, $HET(R^{aF})_{4-9}$, lower alkyl-$HET(R^{aF})_{4-9}$ or lower alkenyl-$HET(R^{aF})_{4-9}$,

each $R^{20F}$ independently is H, lower alkyl, lower alkenyl, lower alkynyl, $CF_3$ or $Ph(R^{13F})_2$,

each $R^{aF}$ independently is a group selected from the followings:

H, OH, a halogen atom, CN, $NO_2$, amino, $C_{1-6}$ alkyl, $C_{2-6}$ alkenyl, $C_{2-6}$ alkynyl, $C_{1-6}$ alkoxy, $C_{2-6}$ alkenyloxy, $C_{2-6}$ alkynyloxy, $C_{1-6}$ alkylamino, di-($C_{1-6}$ alkyl)amino, $CF_3$, C(O)-$C_{1-6}$ alkyl, C(O)-$C_{2-6}$ alkenyl, C(O)-$C_{2-6}$ alkynyl, COOH, COO-$C_{1-6}$ alkyl, COO-$C_{2-6}$ alkenyl and COO-$C_{2-6}$ alkynyl;

in the group, alkyl, alkenyl, alkynyl and alkyl in alkylamino and dialkylamino may be substituted with one to three of the following group(s):

OH, a halogen atom, aryl, $C_{1-6}$ alkoxy, $C_{2-6}$ alkenyloxy, $C_{2-6}$ alkynyloxy, $CF_3$, CO-$C_{1-6}$ alkyl, CO-$C_{2-6}$ alkenyl, CO-$C_{2-6}$ alkynyl, COOH, COO-$C_{1-6}$ alkyl, COO-$C_{2-6}$ alkenyl, COO-$C_{2-6}$ alkynyl, $NH_2$, NH-$C_{1-6}$ alkyl and N-$C_{1-6}$ $alkyl_2$, or a non-toxic salt thereof.

(7) A compound represented by formula (G) mentioned in the specification of WO 00/20371

$$Ar^{1G}\text{-}W^{G}\text{-}Ar^{2G}\text{-}X^{G}\text{-}W^{G} \qquad (G)$$

(in the formula, $Ar^{1G}$ is aryl or heteroaryl and may be substituted with $R^{1G}$ or $R^{3G}$,

$R^{1G}$ is $Y^G{}_{mG}$-$R^{2G}$, $Y^G{}_{mG}$-$Ar^{3G}$, a halogen atom, $N(R^{5G})_2$, CN, $NO_2$, $C(R^{6G})_3$, $CON(R^{5G})_2$, $S(O)_{nG}$ $R^{7G}$ or OH,

$Y^G$ is a connecting chain between $Ar^{1G}$ and $R^{2G}$ or $Ar^{3G}$ and contains 0 to 4 carbon atom(s) and at most one hetero atom selected from O, N and S and the connecting chain may contain CO, $S(O)_{nG}$, -C=C- or acetylene or may be further substituted with $R^{2G}$,

$m^G$ is 0 or 1,

$n^G$ is 0, 1 or 2,

$R^{2G}$ is H, F, $CHF_2$, $CF_3$, lower alkyl or hydroxy-$C_{1-6}$ alkyl, or two $R^{2G}$ may be joined together and represent a at most six membered carbon ring which may contain at most one hetero atom selected from O, N and S,

$Ar^{3G}$ is aryl or heteroaryl which may be substituted with $R^{3G}$,

$R^{3G}$ is $R^{4G}$, a halogen atom, halo-$C_{1-6}$ alkyl, $N(R^{5G})_2$, CN, $NO_2$, $C(R^{6G})_3$, $CON(R^{5G})_2$, $OR^{4G}$, $SR^{4G}$ or $S(O)_{nG}R^{7G}$,

$R^{4G}$ is H, lower alkyl, lower alkenyl, lower alkynyl, $CHF_2$ or $CF_3$,

$R^{5G}$ is $R^{4G}$, phenyl or benzyl, or two $R^{5G}$ in combination with a at most six membered ring containing carbon atoms and at most two hetero atom(s) selected from O, N and S,

$R^{6G}$ is H, F, $CF_3$ or lower alkyl, or two $R^{6G}$ may be taken together and represent a at most six membered ring containing carbon atoms and 0 to 2 hetero atom(s) selected from O, N and S,

$R^{7G}$ is lower alkyl, lower alkenyl, lower alkynyl, $CHF_2$, $CF_3$, $N(R^{5G})_2$, $Ph(R^{8G})_2$ or $CH_2Ph(R^{8G})_2$,

$R^{8G}$ is $R^{4G}$, $OR^{4G}$, $SR^{4G}$ or a halogen atom

$W^G$ is a three- to six-membered connecting chain containing 0 to 2 hetero atom(s) selected from O, N and S and the connecting chain may contain CO, $S(O)_{mG}$, C=C and acetylene and may be further substituted with $R^{9G}$,

$R^{9G}$ is $R^{2G}$, lower alkenyl, lower alkynyl, $OR^{4G}$ or $SR^{4G}$,

$Ar^{2G}$ is aryl or heteroaryl which may be substituted with $R^{3G}$,

$R^{10G}$ is $R^{4G}$, a halogen atom, $N(R^{5G})2$, CN, $NO_2$, $C(R^{6G})3$, $OR^{4G}$, $SR^{4G}$ or $S(O)_{nG}R^{7G}$,

$X^G$ is a connecting group which is substituted at the ortho position to $Ar^{2G}$ based on $W^G$ and it contains 0 to 4 carbon atom(s) and at most one hetero atom selected from O, N and S, may contain CO, $S(O)_{nG}$, C=C or acetylene, and may be further substituted with $R^{11G}$,

$R^{11G}$ has the same meaning as $R^{9G}$,

$Q^G$ is a group selected from COOH, tetrazole, $SO_3H$, hydroxamic acid, $CONHSO_2R^{12G}$ and $SO_2NHCOR^{12G}$,

$R^{12G}$ is a group selected from $CF_3$, lower alkyl, lower alkenyl, lower alkynyl and $Z^9Ar^{4G}$,

$Z^G$ is 0 to 4 connecting chain(s) which may be substituted with $R^{13G}$,

$R^{13G}$ has the same meaning as $R^{9G}$,

$Ar^{4G}$ is aryl or heteroaryl which may be substituted with $R^{14G}$,

$R^{14G}$ is $R^{10G}$ or NHCOMe.), or a non-toxic salt thereof.

Besides the above, the followings are used as $EP_1$ antagonists. They are:

(8) 2,3,6-substituted-4-pyrone derivatives mentioned in the specification of US 4132847,
(9) N-alkenyl-3-hydrobenzo[b]thiophene-2-carboxyamide derivatives mentioned in the specification of EP 160408,
(10) dibenzoxepinecarboxylic acid hydrazide derivatives mentioned in the specification of EP 193822,
(11) 8-chlorodibenzoxazepine-10-carboxylic acid hydrazide derivatives mentioned in the specification of EP 218077,
(12) cyclohept[b]indolealkane derivatives mentioned in the specification of US 4775680,
(13) 9-benzyldifluorotetrahydrocarbazolylacetic acid derivatives mentioned in the specification of EP 300676,
(14) tricyclic hetero ring derivatives mentioned in the specification of EP 480641,
(15) dibenzoxazepine derivatives mentioned in the specification of EP 512399,
(16) dibenzoxazepine derivatives mentioned in the specification of EP 512400,
(17) tricyclic hetero ring derivatives mentioned in the specification of EP 534667,
(18) 10-acyldibenzoxazepine, thiazepine or diazeptine derivative mentioned in the specification of WO 93/07132,

(19) dibenzoxazepine derivative mentioned in the specification of EP 539977,
(20) dibenzoxazepine or dibenzothiazepine derivatives mentioned in the specification of WO 93/13082,
(21) dibenzoxazepinecarboxylic acid derivatives mentioned in the specification of US 5281590,
(22) N-carbazylbenzoxazepine derivative mentioned in the specification of US 530464,
(23) dibenzoxazepine derivatives mentioned in the specification of US 5324722,
(24) dibenzthia- or oxaazepinyl-3-cyclobutene-1,2-dione derivatives mentioned in the specification of US 5354746,
(25) dibenzoxazepine or dibenzothiazepine derivatives mentioned in the specification of US 5354747,
(26) dibenzoxazepine or dibenzothiazepine derivatives mentioned in the specification of US 5420270,
(27) azepine derivatives mentioned in the specification of US 5441950,
(28) dibenzoxazepine derivatives mentioned in the specification of EP 694546,
(29) o-arylmethoxy-arylmethylamino aromatic acid derivatives mentioned in the specification of WO 96/03380,
(30) dibenzoxazpine or dibenzothiazepine derivative mentioned in the specification of US 5504077,
(31) aromatic derivatives mentioned in the specification of WO 96/11902,
(32) o-substituted aromatic compounds mentioned in the specification of EP 752421 and
(33) aromatic derivatives mentioned in the specification of WO 97/00864.

The compounds mentioned in the above (1) to (33) may be converted to pharmaceutically acceptable salts by a known method. The salts are preferably to be pharmaceutically acceptable water-soluble ones.

Examples of appropriate salts are salt of alkaline metal (such as potassium and sodium), salt of alkaline earth metal (such as calcium and magnesium), ammonium salt (such as tetramethylammonium) and salt of pharmaceutically acceptable organic amine (such as triethylamine, methylamine, dimethylamine, cyclopentylamine, benzylamine, phenethylamine, piperidine, monoethanolamine, diethanolamine, tris(hydroxymethyl)methylamine, lysine, arginine and N-methyl-D-glucamine).

Preferable acid addition salts are pharmaceutically acceptable and water-soluble ones. Examples of appropriate acid addition salt are inorganic acid salts such as hydrochloride, hydrobromide, sulfate, phosphate and nitrate and organic acid salts such as acetate, trifluoroacetate, lactate, tartrate, oxalate, fumarate, maleate, citrate, benzoate, methanesulfonate, ethanesulfonate, benzenesulfonate, toluenesulfonate, isethionate, glucuronate and gluconate.

In addition, the compounds of the present invention and salts thereof may be converted to hydrates by a known method.

Specific compounds used in the present invention are the specific compounds mentioned in WO 98/27053, EP 878465, WO 92/19617, WO 96/06822, WO 97/00863, WO 99/47497, WO 00/20371, US 4132847, EP 160408, EP 193822, EP 218077, US 4775680, EP 300676, EP 480641, EP 512399, EP 512400, EP 534667, WO 93/07132, EP 539977, WO 93/13082, US 5281590, US 530646, US 5324722, US 5354746, US 5354747, US 5420270, US 5441950, EP 694546, WO 96/03380, US 5504077, WO 96/11902, EP 752421 and WO 97/00864, such as the compounds mentioned in Examples thereof.

Among the compounds mentioned in the above specification, preferred ones are the compounds which bind to an $EP_1$ receptor showing an antagonism and more preferably, the compounds which specifically bond to an $EP_1$ receptor showing an antagonism.

Still more preferred ones are 6-[(2S,3S)-3-(4-chloro-2-methylphenylsulfonylaminomethyl)-bicyclo[2.2.2]octan-2-yl]-5Z-hexenoic acid (the compound mentioned in Example 2C of the specification of EP 878465), 8-chlorodibenz [b,f][1.4]oxazepine-10(11H)-carboxylic acid 2-[1-oxo-3-(4-pyridinyl)propyl]hydrazide·monohydrochloride (the compound mentioned in Example 44 in the specification of WO 92/19617) and N-(3,5-dimethylisoxazol-4-ylsulfonyl)-6-[N-(5-chloro-2-(isobutyloxy)benzyl)-N-ethylamino]pyridazine-3-carboxamide (the compound mentioned in the first paragraph in Example 8 of the specification of WO 97/00863).

The compounds shown in the above (1) to (33) are able to be manufactured by the method mentioned in each of the corresponding specifications of the International Publications, U. S. Patents or European Publications.

Best Mode for Carrying Out the Invention

Although efficacy of the compounds having an $EP_1$ receptor antagonism to pollakiuria was proved by the following experiments, the present invention is not limited thereto. Compounds of the present invention are not limited to the substances having specific chemical structures but all compounds having an $EP_1$ antagonism are included within the scope of the present invention.

Example 1: Effect of an $EP_1$ antagonist to pathologic pollakiuria induced by sulprostone

(i) Experiments on suppression of an increase in urinated amount and frequency of rats induced by sulprostone

Urinary frequency and urinated amount were measured by a measuring apparatus for urinated amount (K. K. Neuroscience) using male rats of a CD (SD) IGS strain.

Compound of the present invention (4 mL/kg) was administered orally, and after 30 minutes, sulprostone (200 μg/4mL/kg) was subcutaneously administered. Urinary frequency and amount were measured during the period of from administration of sulprostone until 3 hours after administration. Suppressing rate (%) of the frequency for each compound was determined by the following formula.

Suppressing rate (%) = {[(A group to which the vehicle was administered) - (a sham group)] - [(A group to which the compound of the present invention was administered ) - (A sham group)]}/{(A group to which the vehicle was administered) - (A sham group)} × 100

Suppressing rate of frequency by each compound in pollakiuria model rats induced by sulprostone is shown in Table 1.

(ii) Experiment of suppression of an increase in urinary frequency in rats induced by acetic acid

Abdomen of a rat was subjected to a midline incision under anesthetization with pentobarbital (50 mg/kg, i. p.) and bladder was exposed. Urine in the bladder was removed using a syringe equipped with an intraocular injection needle (30G × 314). After that, 0.5 ml of 1% acetic acid solution was injected into the bladder using a syringe equipped with an intraocular injection needle (30G × 3/4) and injury was closed.

With regard to measurement of urination of the animal, the animal was placed in a metabolism cage and urination was recorded with the lapse of time using a urination measuring apparatus (K. K. Neuroscience).

When acetic acid was injected into bladder of rat, urination frequency increased continuously and pollakiuria where each urinated amount decreased was induced. Each of the test drugs was evaluated using the stimulated bladder.

Evaluation of the test drug was conducted in such a manner that the test drug was administered after 2 days from injection of acetic acid into the bladder and suppressing rates for the frequency increased within 6 hours thereafter was compared. Suppressing rate (%) of frequency by each compound was determined by the following formula.

Suppressing rate (%) = {[(A group to which the vehicle was administered) - (A sham group)] - [(A group to which the compound of the present invention was administered) - (A sham group)]}/{(A group to which the vehicle was administered) - (A sham group)} × 100

Suppressing rate of frequency by each compound in pollakiuria model rats induced by acetic acid is shown in Table 1.

With regard to the test compounds,

(1) 6-[(2S,3S)-3-(4-chloro-2-methylphenylsulfonylaminomethyl)bicyclo[2.2.2]octan-2-yl]-5Z-hexenoic acid (the compound mentioned in Example 2C of the specification of EP 878465)

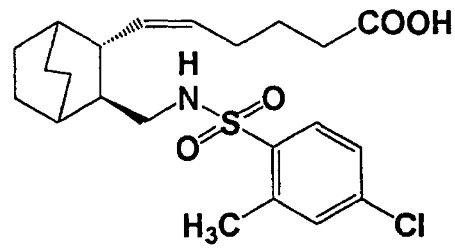

(2) 8-chlorodibenz[b,f][1.4]oxazepine-10(11H)-carboxylic acid·2-[1-oxo-3-(4-pyridinyl)propyl]hydrazide·monohydrochloride (the compound mentioned in Example 44 of the specification of WO 92/19617) and

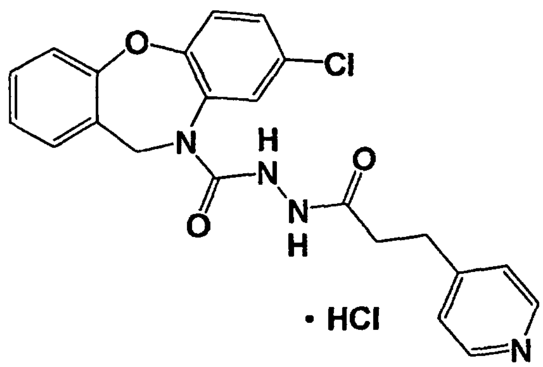

(3) N-(3,5-dimethylisoxazol-4-ylsulfonyl)-6-[N- (5-chloro-2-(isobutyloxy)benzyl)-N-ethylamino]pyridazine-3-carboxamide (the compound mentioned in the first paragraph in Example 8 of the specification of WO 97/00863)

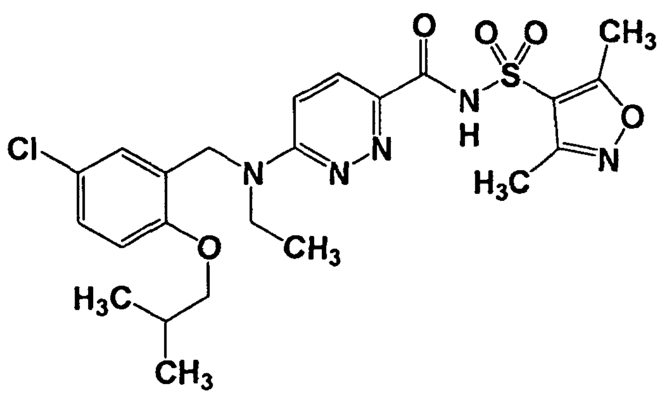

were used.

Table 1: Suppressing Rates of Urinary Frequency in Pollakiuria Models Induced by Sulprostone in Awake Rats and in Pollakiuria Models Induced by Acetic Acid in Awake Rats

Table 1

| Compound | Dose (mg/kg) | Administering Route | Sulprostone-Induced Pollakiuria Model | Acetic Acid-Induced Pollakiuria Model |
|---|---|---|---|---|
| | | | Inhibiting Rate (%) for Urinary Frequency | |
| (1) | 10 | orally | 58.0 ± 12.0 * | 44.3 ± 12.4 * |
| (2) | 10 | subcutaneously | 38.2 ± 4.6 * | - |

Table 1 (continued)

| Compound | Dose (mg/kg) | Administering Route | Sulprostone-Induced Pollakiuria Model | Acetic Acid-Induced Pollakiuria Model |
|---|---|---|---|---|
| | | | Inhibiting Rate (%) for Urinary Frequency | |
| (3) | 3 | subcutaneously | 49.2 ± 2.8 * | - |

(3) Investigation using $EP_1$, $EP_2$, $EP_3$ and $EP_4$ Agonists

Only by an $EP_1$ agonist, the same action as $PGE_2$ was recognized. From the result, it was suggested that $PGE_2$ promotes excitability of parasympathetic ganglion subneuron via an $EP_1$ receptor participating in generation of a long-term excitement.

In vertebra, it has been known that production of $PGE_2$ is promoted by pain stimulation, *etc.* and it is supposed that, even in a state of pollakiuria, there is a possibility of promotion of $PGE_2$ production in vertebra. There is a possibility that excitability of PGN is promoted by $PGE_2$ which is produced by any cause whereupon contraction of bladder increased and an active state in the bladder is induced.

[Discussion]

From the above experimental result, it has been clarified that a compound having an $EP_1$ receptor antagonistic activity significantly shows a suppressive activity for urinary frequency in a model where pollakiuria is inducted. Accordingly, it is likely that other compounds having an $EP_1$ receptor antagonistic activity also suppress the pollakiuria in the same manner.

[Toxicity]

It has been confirmed that toxicity of the compounds of the present invention is sufficiently low and that they are sufficiently safe for the use as agents.

Industrial Applicability

[Application to Drugs]

Compounds having an $EP_1$ receptor antagonistic activity are useful for the treatment and/or the prevention of pollakiuria (that which is due to nurogenic bladder, nervous bladder, stimulated bladder, unstable bladder, benign proststatic hypertrophy, etc.), urinary incontinence and lower uropathy.

The compound represented by formula (I) or a non-toxic salt thereof may also be administered as a concomitant agent in combination with other agents for

1) supplementing and/or reinforcement of preventive and/or treating effect(s) of the compound,
(2) improvement in kinetics and absorption of the compound and reduction of dose
and/or
3) reduction of side effect of the compound.

A concomitant agent of the compound represented by formula (I) with other agents may be administered in a mode of compounded agent in which both components are compounded in a single preparation or in a mode of separate preparations. When administration is conducted using separate preparations, a simultaneous administration and administrations with time difference is included. In the case of administrations with time difference, the compound represented by formula (I) may be firstly administered and then other drug may be administered, or the other drug may be firstly administered and then the compound represented by formula (I) may be administered. Each of the methods for the administration may be same or different.

There is no particular limitation for the diseases for which the above-mentioned concomitant agent achieves the preventive and/or the treating effect but any disease will be acceptable so far as it supplements and/or enforces the preventive and/or the treating effect of the compound represented by the above formula (I).

For example, examples of the other drug for supplementing and/or reinforcing the preventive and/or the treating effect of the compound represented by formula (I) to pollakiuria and urinary incontinence include anticholinergic agent, tricyclic antidepressant, $\alpha_1$ agonist, $\alpha_1$ antagonist, GABA agonist, anti-diuretic, anti-androgenic agent, corpus

luteum hormone, $NK_1$ antagonist, $\beta_3$ agonist, P2X antagonist, potassium channel opener, LPA, $EP_3$ antagonist, capsaicin (resiniferatoxin), 5α-reductase inhibitor and muscarine (M1, M3) antagonist. They also include 5-HT reuptake inhibitor, 5-$HT_{1A}$ antagonist, ACh antagonist, Ca cannel antagonist, H1 blocker, K channel regulator, muscarine (M1) agonist, muscarine (M1, M3) antagonist, NE reuptake inhibitor, neurokinin (NK1, NK2, NK3) antagonist, μ agonist, σ agonist, caspase inhibitor, vasopressin V2 agonist, β3 agonist, dopamine reuptake inhibitor, *etc.*

Examples of the $\alpha_1$ antagonist include terazosin hydrochloride, bunazosin hydrochloride, urapidil, tamsulosin hydrochloride, doxazocin mesilate, prazosin hydrochloride, indoramine, naftopidil, alfuzosin hydrochloride and AIO-8507L.

Examples of the anticholinergic agent include oxybutynin hydrochloride, bethanechol chloride, propiverine hydrochloride, propantheline bromide, methylbenactyzium bromide, butylscopolamine bromide, tolterodine tartrate, trospium chloride, Z-338, K-112166-04, KRP-197, darifenacin and YM-905.

Examples of the 5α-reductase inhibitor include finasteride and GI-998745.

Examples of the antiandrogen include oxendolone, osaterone acetate and bicalutamide.

Examples of the muscarine antagonist include YM 905, KRP 197 and ONO-8025.

Examples of the 5-HT reuptake inhibitor include duloxetine hydrochloride, *etc*.

Examples of the 5-$HT_{1A}$ antagonist include REC-15-3079, *etc*. Examples of the ACh antagonist include oxybutynin, *etc*.

Examples of the Ca channel antagonist include terflavoxate hydrochloride and FK-584.

Examples of the H1 blocker include tekastemizole, levocabastine hydrochloride, astemizole, norastemizole, diphenhydramine and chlorpheniramine maleate.

Examples of the K-channel regulator include NS-4591, ABT-598, AZD-0947, NS-8, YM-934, ZD-6169, WAY-151616 and A-278637.

Examples of the muscarine (M1) agonist include albamelin maleate and fesoterozin.

Examples of the muscarine (M1, M3) antagonist include KRP-197, ONO-8025, vamicamide, tolterodine tartrate, trospium chloride, J-104135, solifenacin succinate, darifenacin, YM-35636 and UFA-0272.

Examples of the NE reuptake inhibitor include S-didesmethyl sibutramine, *etc*.

Examples of the neurokinin (NK1, NK2, NK3) antagonist include TAK-637, SSR-240600, AZD-5106 and talnetant.

Examples of the vasopressin V2 agonist include OPC-51803, WAY-141608, FE-106483 and VNA-932.

Examples of the α1 agonist include SL-251039, midodrine hydrochloride and ABT-866.

Examples of the β3 agonist include KUC-7483, *etc*.

Examples of the dopamine reuptake inhibitor include S-didesmethyl sibutramine, etc.

There is no particular limitation for the ratio by weight of the compound represented by formula (I) to other agent.

With regard to other agents, two or more members of any agent may be administered in combination.

Such other agents which supplement and/or reinforce the preventive and/or the treating effect of the compound represented by formula (I) include not only those which have been found on the basis of the above-mentioned mechanism but also those which will be found in future.

When the compound represented by formula (I) used in the present invention, an ester or non-toxic salt thereof or a concomitant agent thereof with other agents is used for the above-mentioned purpose, it is usually administered either systemically or locally in an oral or a parenteral form.

Although the dose varies depending upon age, body weight, symptom, treating effect, administration method, treating time, *etc.*, it is usually within a range of 1 mg to 1,000 mg for one administration for an adult and that is orally administered one to several times a day or it is usually within a range of 1 mg to 100 mg per administration per adult and that is parenterally (preferably intravenously) administered one to several times a day or is intravenously administered continuously within a range of 1 to 24 hour(s) a day.

Needless to say, the dose varies according to various conditions as mentioned hereinabove and, therefore, there are some cases where less amount than the above will be sufficient while there are some other cases where more amount than the above will be necessary.

When the compound represented by formula (I), a non-toxic salt thereof or a concomitant agent of the compound represented by formula (I) with other drug is administered, it is used as a solid composition, a liquid composition or other compositions for oral administration or is used as an injection agent, an agent for external application, a suppository, *etc.* for parenteral administration.

A solid composition for oral administration includes tablets, pills, capsules, diluted powder, granules, *etc*.

A capsule composition includes hard capsules and soft capsules.

In such a solid composition, one or more active ingredient(s) is/are mixed with at least one inert diluent such as lactose, mannitol, glucose, hydroxypropyl cellulose, finely crystalline cellulose, starch, polyvinylpyrrolidone and magnesium alminometasilicate. The composition may also contain an additive which is other than the inert diluent of lubricant such as magnesium stearate), disintegrating agent such as calcium cellulose glycolate, stabilizer such as lactose and solubilizing agent such as glutamic acid and aspartic acid in accordance with a conventional method. If necessary, tablets and pills may be coated with film of an intragastically soluble or enterically soluble substance such as sugar, gelatin, hydroxypropylcellulose or hydroxypropylmethylcellulose phthalate, or may be coated with two or more layers. Capsules of a substance such as gelatin which is able to be absorbed may be included therein.

A liquid composition for oral administration includes pharmaceutically acceptable emulsion, solution, syrup, elixir, *etc*. In such a liquid composition, one or more active substance(s) is/are contained in a commonly used inert diluent (such as pure water and ethanol). In addition to the inert diluent, the composition may also contain auxiliary agent such as wetting agent, suspending agent, sweetener, flavor, aromatic agent or antiseptic.

Other compositions for oral administration include a spray agent which contains one or more active substance (s) and is formulated by a method known *per se*. In addition to the inert diluent, the said composition may also contain a stabilizer such as sodium bisulfite and a buffer giving an isotonic property such as an isotonizing agent (e.g., sodium chloride, sodium citrate or citric acid). A method for the manufacture of the spray agent is mentioned, for example in U. S. Patent No. 2,868,691 and U. S. Patent No. 3,095,355 in detail.

The injection agent for parenteral administration according to the present invention includes aseptic aqueous and/or non-aqueous solution, suspension and emulsion. The aqueous solution and suspension include, for example, distilled water for injection and a physiological saline solution. With regard to the non-aqueous solution and suspension, there are, for example, propylene glycol, polyethylene glycol, vegetable oil such as olive oil, alcohol such as ethanol and Polysolvate 80 (registered trade mark). It is also possible to use by mixing aseptic aqueous and non-aqueous solutions, suspensions and emulsions. Such a composition may further contain an auxiliary agent such as antiseptic, wetting agent, emulsifier, dispersing agent, stabilizer such as lactose and solubilizing aid such as glutamic acid and aspartic acid. They are made aseptic by means of filtration passing through a bacteria-retaining filter, compounding with a bactericide or irradiation. It is also possible to use in such a manner that an aseptic solid composition is manufactured and, before the use of the freeze-dried substance for example, it is dissolved in distilled water for injection which is made aseptic or is aseptic or in other solvent.

Other composition for parenteral administration includes outer solution preparation, ointment, liniment, suppository for intrarectal administration, pessary for intravaginal administration.

[Example for Preparing Pharmaceutical Preparation]

The following components were mixed by a conventional method and made into tablets to give 100 tablets each containing 50 mg of active ingredient.

| | |
|---|---|
| 6-[(2S,3S)-3-(4-Chloro-2-methylphenylsulfonylaminomethyl)bicycle[2.2.2]-octan-2-yl]-5Z-hexenoic acid | 5.0 g |
| Carboxymethyl cellulose calcium | 0.2 g |
| Magnesium stearate | 0.1 g |
| Finely crystalline cellulose | 4.7 g |

## Claims

1. An agent for the treatment and/or prevention of pollakiuria comprising a compound having an antagonism to an $EP_1$ receptor which is a prostaglandin $E_2$ receptor.

2. The agent for the treatment and/or prevention of pollakiuria according to claim 1, wherein the compound having an antagonism to an $EP_1$ receptor is selected from

    (1) the compounds mentioned in the specification of WO 98/27053,
    (2) the compounds mentioned in the specification of EP 878465,
    (3) the compounds mentioned in the specification of WO 92/19617,
    (4) the compounds mentioned in the specification of WO 96/06822,
    (5) the compounds mentioned in the specification of WO 97/00863,
    (6) the compounds mentioned in the specification of WO 99/47497,
    (7) the compounds mentioned in the specification of WO 00/20371,
    (8) the compounds mentioned in the specification of US 4132847,
    (9) the compounds mentioned in the specification of EP 160408,
    (10) the compounds mentioned in the specification of EP 193822,

(11) the compounds mentioned in the specification of EP 218077,
(12) the compounds mentioned in the specification of US 4775680,
(13) the compounds mentioned in the specification of EP 300676,
(14) the compounds mentioned in the specification of EP 480641,
(15) the compounds mentioned in the specification of EP 512399,
(16) the compounds mentioned in the specification of EP 512400,
(17) the compounds mentioned in the specification of EP 534667,
(18) the compounds mentioned in the specification of WO 93/07132,
(19) the compounds mentioned in the specification of EP 539977,
(20) the compounds mentioned in the specification of WO 93/13082,
(21) the compounds mentioned in the specification of US 5281590,
(22) the compounds mentioned in the specification of US 530646,
(23) the compounds mentioned in the specification of US 5324722,
(24) the compounds mentioned in the specification of US 5354746,
(25) the compounds mentioned in the specification of US 5354747,
(26) the compounds mentioned in the specification of US 5420270,
(27) the compounds mentioned in the specification of US 5441950,
(28) the compounds mentioned in the specification of EP 694546,
(29) the compounds mentioned in the specification of WO 96/03380,
(30) the compounds mentioned in the specification of US 5504077,
(31) the compounds mentioned in the specification of WO 96/11902,
(32) the compounds mentioned in the specification of EP 752421 and
(33) the compounds mentioned in the specification of WO 97/00864.

**3.** The agent for the treatment and/or prevention of pollakiuria according to claim 1, represented by formula (A) mentioned in the specification of WO 98/27053

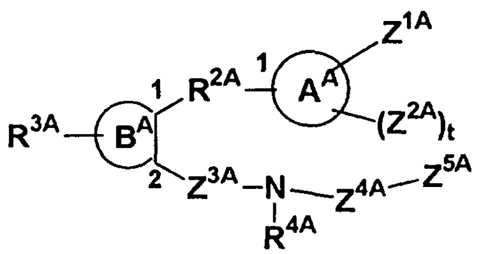

(A)

(in the formula,

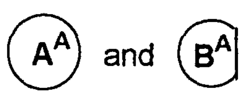

each independently is a $C_{5-15}$ carbon ring or a five- to seven-membered hetero ring having 1 or 2 oxygen, sulfur or nitrogen atom(s),

$Z^{1A}$ is

-$COR^{1A}$,
-$C_{1-4}$ alkylene-$COR^{1A}$,
-$CH=CH-COR^{1A}$,
-$C\equiv C-COR^{1A}$ or
-$O-C_{1-3}$ alkylene-$COR^{1A}$

(in each formula, $R^{1A}$ is a hydroxyl group, $C_{1-4}$ alkoxy or a group represented by a formula $NR^{6A}R^{7A}$ (in the formula, $R^{6A}$ and $R^{7A}$ each independently is a hydrogen atom or $C_{1-4}$ alkyl) or -$C_{1-5}$ alkylene-OH,

$Z^{2A}$ is a hydrogen atom, $C_{1-4}$ alkyl, $C_{1-4}$ alkoxy, nitro, halogen, trifluoromethyl, trifluoromethoxy, hydroxyl group or a group represented by the formula $COR^{1A}$ (in the formula, $R^{1A}$ has the same meaning as defined above),

$Z^{3A}$ is a single bond or $C_{1-4}$ alkylene,

$Z^{4A}$ is $SO_2$ or CO,
$Z^{5A}$ is

(1) $C_{1-8}$ alkyl, $C_{2-8}$ alkenyl, or $C_{2-8}$ alkynyl,
(2) phenyl, $C_{3-7}$ cycloalkyl, a five- to seven-membered hetero ring having one or two oxygen, sulfur or nitrogen atom(s),
(3) phenyl or $C_{3-7}$ cycloalkyl-substituted $C_{1-4}$ alkyl, $C_{2-4}$ alkenyl or $C_{2-4}$ alkynyl,

(in the above (2) and (3), phenyl, $C_{3-7}$ cycloalkyl and a five- to seven-membered hetero ring having one or two oxygen, sulfur or nitrogen atom(s) may be substituted with one to five $R^{5A}$ group(s) (each of plural $R^{5A}$ independently is a hydrogen atom, $C_{1-6}$ alkyl, $C_{1-6}$ alkoxy, $C_{1-6}$ alkylthio, nitro, halogen, trifluoromethyl, trifluoromethoxy or a hydroxyl group)),
$R^{2A}$ is
$CONR^{8A}$,
$NR^{8A}CO$,
$CONR^{8A}$-$C_{1-4}$ alkylene,
$C_{1-4}$ alkylene-$CONR^{8A}$,
$NR^{8A}CO$-$C_{1-4}$ alkylene,
$C_{1-4}$ alkylene-$NR^{8A}CO$,
$C_{1-3}$ alkylene-$CONR^{8A}$-$C_{1-3}$ alkylene,
$C_{1-3}$ alkylene-$NR^{8A}CO$-$C_{1-3}$ alkylene
(in each formula, $R^{8A}$ is a hydrogen atom or $C_{1-4}$ alkyl),
O, S, $NZ^{6A}$
(in the formula, $Z^{6A}$ is a hydrogen atom or $C_{1-4}$ alkyl),
$Z^{7A}$-$C_{1-4}$ alkylene,
$C_{1-4}$ alkylene-$Z^{7A}$,
$C_{1-3}$ alkylene-$Z^{7A}$-$C_{1-3}$ alkylene
(in the formulae, $Z^{7A}$ is O, S or a group represented by the formula $NZ^{6A}$ (in the formula, $Z^{6A}$ has the same meaning as defined above)),
CO,
CO-$C_{1-4}$ alkylene,
$C_{1-4}$ alkylene-CO,
$C_{1-3}$ alkylene-CO-$C_{1-3}$ alkylene,
$C_{2-4}$ alkylene,
$C_{2-4}$ alkenylene or
$C_{2-4}$ alkynylene
$R^{3A}$ is a hydrogen atom, $C_{1-6}$ alkyl, $C_{1-6}$ alkoxy, $C_{1-6}$ alkylthio, nitro, halogen, trifluoromethyl, trifluoromethoxy, hydroxyl group or hydroxymethyl,
$R^{4A}$ is

(1) a hydrogen atom,
(2) $C_{1-8}$ alkyl, $C_{2-8}$ alkenyl or $C_{2-8}$ alkynyl,
(3) $C_{1-6}$ alkyl substituted with one or two group(s) selected from the group consisting of $COOZ^{8A}$, $COZ^{9A}Z^{10A}$, $OZ^{8A}$ group (in each group, $Z^{8A}$, $Z^{9A}$ and $Z^{10A}$ each independently is a hydrogen atom or $C_{1-4}$ alkyl) and $C_{1-4}$ alkoxy-$C_{1-4}$ alkoxy,
(4) $C_{3-7}$ cycloalkyl,
(5) phenyl or $C_{3-7}$ cycloalkyl-substituted $C_{1-4}$ alkyl, $C_{2-4}$ alkenyl or $C_{2-4}$ alkynyl,

(phenyl and $C_{3-7}$ cycloalkyl in the above (4) and (5) may be substituted with one to five $R^{5A}$ group(s) ($R^{5A}$ has the same meaning as defined above)) and
$n^A$ and $t^A$ each independently is an integer of 1 to 4,
wherein

(1) $R^{2A}$ and $Z^{3A}$ each binds to only 1- and 2-position of

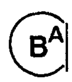

and

(2) when

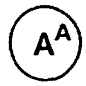

is a benzene ring and $(Z^{2A})_t^A$ is not $COR^{1A}$, then $Z^{1A}$ binds to only 3- or 4-position of the benzene ring.), wherein it is a sulfonamide or carboamide derivative or a non-toxic salt thereof.

**4.** The agent for the treatment and/or prevention of pollakiuria according to claim 1, represented by formula (B) mentioned in the specification of EP878465

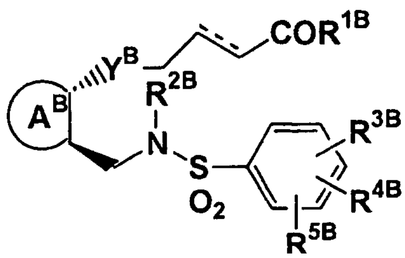

(B)

(in the formula,

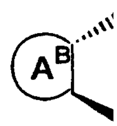

is a group represented by

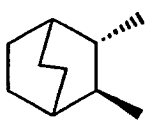
(a)

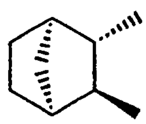
(b)

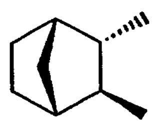
(c)

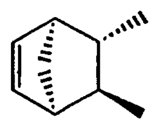
(d)

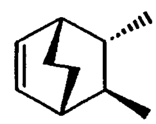
(e)

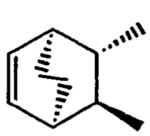
(f)

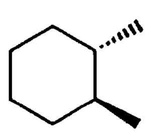
(g)

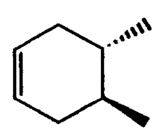
(h)

or

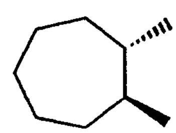
(i)

$R^{1B}$ is hydroxy, a $C_{1-4}$ alkoxy group or a group represented by formula $NR^{6B}N^{7B}$ (in the formula, $R^{6B}$ and $R^{7B}$ each independently is a hydrogen atom or a $C_{1-4}$ alkyl group),
$R^{2B}$ is a hydrogen atom or a $C_{1-4}$ alkyl group,
$R^{3B}$ and $R^{4B}$ each is a $C_{1-4}$ alkyl group, a halogen atom or trifluoromethyl group,
$R^{5B}$ is a hydrogen atom, a $C_{1-4}$ alkyl group, a halogen atom or trifluoromethyl group,
$Y^B$ is cis-vinylene or trans-vinylene and

a symbol 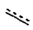 is a single bond or a double bond, whereinwhen

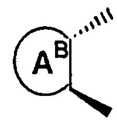

is a formula

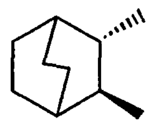

$R^{1B}$ is hydroxy or a $C_{1-4}$ alkoxy group, $R^{2B}$ is a hydrogen atom, $Y^B$ is cis-vinylene and the symbol 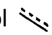 is a single bond, then

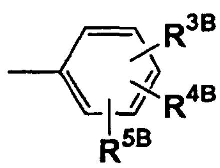 is not 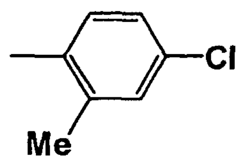 .),

wherein it is a benzenesulfonamide derivative, a non-toxic salt thereof or a cyclodextrin clathrate.

**5.** The agent for the treatment and/or the prevention of pollakiuria according to claim 1, wherein it is a compound represented by formula (C) mentioned in the specification of WO 92/19617

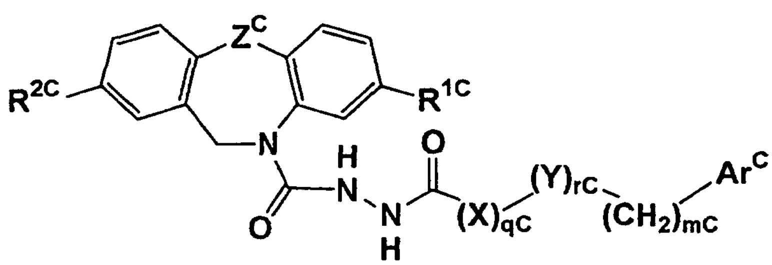

(C)

(in the formula, $R^{1C}$ is a hydrogen atom, halogen or $-CF_3$;
$R^{2C}$ is a hydrogen atom, halogen, -OH or $-OCH_3$;
$Z^C$ is -O-, -S-, -S(O)- or $-S(O)_2-$;
$X^C$ is -CH=CH-, $-CF_2-$, -CHF-, $-(CH_2)_{nC}-$ or $-(CH_2)_{pC}$-CH=CH-;
$Y^C$ is -CH(OH)-, $-NR^{3C}-$, -S-, -S(O)-, $-S(O)_2-$ or -O-;
$q^C$ is 0 or 1;
$r^C$ is 0 or 1 (wherein when

(1) $X^C$ is -CH=CH-, $-(CH_2)_{nC}-$ or $-(CH_2)_{pC}$-CH=CH-, $q^C$ is 1 and $Ar^C$ is imidazole or phenyl,
(2) X is $-(CH_2)_{nC}$, $q^C$ is 1, $n^C$ is 1 and $Ar^C$ is halogen, methyl or alkoxy-substituted ethylphenyl or
(3) $q^C$ is 1, $m^C$ is 1, 2, 3, 4, 5 or 6 and $Ar^C$ is imidazole or phenyl, then $r^C$ is not 0);

$m^C$ is 0 to 6 (wherein when xC is $-(CH_2)_{nC}-$, $q^C$ is 1, $Y^C$ is -O-, -S-, -S(O)- or $-S(O)_2-$ and $Ar^C$ is phenyl, then $m^C$ is not 0);
$n^C$ is an integer of 1 to 6;
$p^C$ is an integer of 1 to 6;
$R^{3C}$ is a hydrogen atom or tert-butyloxycarbonyl and

$Ar^C$ is aryl, alkyl-substituted aryl or aryl-substituted aryl).

**6.** A compound represented by formula (D) mentioned in the specification of WO 96/06822

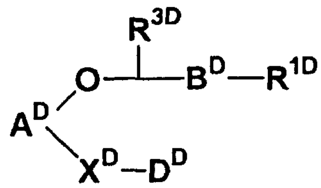

(D)

(except for 4-(2-benzyl-3-hydroxy-4-formylphenoxymethyl)-3- methoxybenzoic acid and 4-(2-(3-phenylprop-2-en-1-yl)-3- hydroxy-4-formylphenoxymethyl-3-methoxybenzoic acid)

(in the formula, $A^D$ is optionally-substituted eight- to ten-membered bicyclic heteroaryl, five- or six-membered heteroaryl, naphthyl or phenyl where the binding groups -OCH($R^{3D}$)- and -$X^D$ - are positioned at 1- and 2-positions each other on a cyclic carbon atoms,

$B^D$ is an optionally-substituted five- or six-membered heteroaryl ring or optionally-substituted phenyl,

$D^D$ is optionally substituted pyridyl, pyrazinyl, pyrimidyl, pyridazinyl, pyrrolyl, thienyl, furyl, pyrazolyl, thiazolyl, isothiazolyl, oxazolyl, isoxazolyl or phenyl,

$X^D$ is a formula $-(CHR^{4D})_{nD}-$ or $-(CHR^{4D})_{pD}CR^{4D}=CR^{4D}(CHR^{4D})_{qD}-$ in which $n^D$ is 1 to 3 and both $p^D$ and $q^D$ are 0 or one of $p^D$ and $q^D$ is 1 while another is 0,

$R^{1D}$ is positioned on the ring $B^D$ in a relation of 1,3 or 1,4 with a binding group -OCH($R^{3D}$)- on a six-membered ring or in a relation of 1,3-with a binding group -OCH($R^{3D}$)- on a five-membered ring and is carboxy, carboxy-$C_{1-3}$ alkyl, tetreazolyl, tetrazolyl-$C_{1-3}$ alkyl, tetronic acid, hydroxamic acid or sulfonic acid, or $R^{1D}$ is a formula $-CONR^{aD}R^{a1D}$ in which $R^{aD}$ is a hydrogen atom or $C_{1-6}$ alkyl, $R^{a1}$ is a hydrogen atom or optionally substituted $C_{1-6}$ alkyl, $C_{2-6}$ alkenyl, $C_{2-6}$ alkynyl, $C_{3-7}$ cycloalkyl, $C_{3-7}$ cycloalkyl-$C_{1-6}$ alkyl, $C_{3-7}$ cycloalkyl-$C_{2-6}$ alkenyl, $C_{3-7}$ cycloalkyl-$C_{2-6}$ alkynyl, $C_{5-7}$ cycloalkenyl, $C_{3-7}$ cycloalkenyl-$C_{1-6}$ alkyl, $C_{5-7}$ cycloalkenyl-$C_{2-6}$ alkenyl, $C_{5-7}$ cycloalkenyl-$C_{2-6}$ alkynyl, $C_{1-3}$ alkyl which is substituted with a five- or six-membered saturated or a partially saturated hetero ring, five- or six-membered saturated or a partially saturated hetero ring or five- or six-membered heteroaryl or, in the formula, $R^{aD}$ and $R^{a1D}$ form an amino acid residue or ester thereof together with an amide nitrogen ($NR^{aD}R^{a1D}$) to which they bind, or $R^{1D}$ is a formula $-CONHSO_2R^{bD}$ in which $R^{bD}$ is optionally substituted $C_{1-6}$ alkyl, $C_{2-6}$ alkenyl, $C_{2-6}$ alkynyl, $C_{3-7}$ cycloalkyl-$C_{1-6}$ alkyl, $C_{3-7}$ cycloalkyl-$C_{2-6}$ alkenyl, $C_{3-7}$ cycloalkyl-$C_{2-6}$ alkynyl, $C_{3-7}$ cycloalkenyl-$C_{1-6}$ alkyl, $C_{3-7}$ cycloalkenyl-$C_{2-6}$ alkenyl, $C_{3-7}$ cycloalkenyl-$C_{2-6}$ alkynyl, five- or six-membered heteroaryl, five- or six-membered heteroaryl-$C_{1-6}$ alkyl, phenyl, phenyl-$C_{1-6}$ alkyl, five- or six-membered saturated or a partially saturated hetero ring or five- or six-membered saturated or a partially saturated hetero ring-$C_{1-6}$ alkyl,

$R^{3D}$ is a hydrogen atom or $C_{1-4}$ alkyl and

$R^{4D}$ is a hydrogen atom or $C_{1-4}$ alkyl), or an N-oxide thereof in case chemically possible, a sulfur oxide having ring in case chemically possible, a pharmaceutically acceptable salt thereof, or an ester or amide hydrolyzable in the living body.

**7.** The agent for the treatment and/or the prevention of pollakiuria according to claim 1, wherein it is a compound of formula (E) mentioned in the specification of WO97/00863

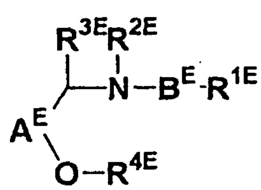

(E)

(in the formula $A^E$ is the following which is optionally substituted:

phenyl, naphthyl, pyridyl, pyrazinyl, pyridazinyl, pyrimidyl, thienyl, thiazolyl, oxazolyl or thiadiazolyl having at least two adjacent ring carbon atoms;

in that case, -CH($R^{3E}$)N($R^{2E}$)$B^E$-$R^{1E}$ and -$OR^{4E}$ are positioned at 1 and 2 each other on the ring carbon atoms and the ring atom positioned at ortho to an $OR^{4E}$ binding group (and, therefore, at 3-position on the basis of a -$CHR^{3E}NR^{2E}$- binding group) is not substituted;

$B^E$ is the following which is optionally substituted:

phenyl, pyridyl, thiazolyl, oxazolyl, thienyl, thiadiazolyl, imidazolyl, pyrazinyl, pyridazinyl or pyrimidyl; $R^{1E}$ is positioned 1,3 or 1,4 to a -CH($R^{3E}$)N($R^{2E}$)- binding group on a ring $B^E$ and $R^{1E}$ is carboxy, carboxy-$C_{1-3}$ alkyl, tetrazolyl, tetrazoly-$C_{1-3}$ alkyl, tetronic acid, hydroxamic acid or sulfonic acid or $R^{1E}$ is -$CONR^{aE}R^{a1E}$ [in that case, $R^{aE}$ is a hydrogen atom or $C_{1-6}$ alkyl and $R^{a1E}$ is a hydrogen atom, $C_{1-6}$ alkyl (in some cases, it is substituted with halogen, amino, $C_{1-4}$ alkylamino, di-$C_{1-4}$ alkylamino, hydroxy, nitro, cyano, trifluoromethyl, $C_{1-4}$ alkoxy or $C_{1-4}$ alkoxycarbonyl), $C_{2-6}$ alkenyl (in that case, a double bond is not at 1-position), $C_{2-6}$ alkynyl (in that case, a triple bond is not at 1-position), carboxyphenyl, five-or six-membered heterocyclic $C_{1-3}$ alkyl, five- or six-membered heteroaryl $C_{1-3}$ alkyl, five- or six-membered heterocyclic or five- to six-membered heteroaryl or $R^{aE}$ and $R^{a1E}$ form an amino acid residue or ester thereof together with an amide nitrogen ($NR^{AE}$ $R^{a1E}$) to which they bind] or $R^1$ is a group of formula -$CONHSO_2R^b$ [in that case, $R^{bE}$ is $C_{1-6}$ alkyl (in some cases, it may be substituted with halogen, hydroxy, nitro, cyano, trifluoromethyl, $C_{1-4}$ alkoxy, amino, $C_{1-4}$ alkylamino, di-$C_{1-4}$ alkylamino or $C_{1-4}$ alkoxycarbonyl), $C_{2-6}$ alkenyl (in that case, a double bond is not at 1-position), $C_{2-6}$ alkynyl (in that case, a triple bond is not at 1-position), five- or six-membered heterocyclic $C_{1-3}$ alkyl, five- or six-membered heteroaryl $C_{1-3}$ alkyl, five- or six-membered heterocyclic, five- or six-membered heteroaryl or phenyl];

in that case, any heterocyclic or heteroaryl group in $R^{a1E}$ is optionally substituted with halogen, hydroxy, nitro, hydroxy, amino, cyano, $C_{1-6}$ alkoxy, $C_{1-6}$ alkyl $S(O)_p{}^E$- ($p^E$ is 0, 1 or 2), $C_{1-6}$ alkylcarbamoyl, $C_{1-4}$ alkylcarbamoyl, di-($C_{1-4}$ alkyl)carbamoyl, $C_{2-6}$ alkenyl, $C_{2-6}$ alkynyl, $C_{1-4}$ alkoxycarbonylamino, $C_{1-4}$ alkanoylamino, $C_{1-4}$ alkanoyl)(N-$C_{1-4}$ alkyl)amino, $C_{1-4}$ alkanesulfonamido, benzenesulfonamido, aminosulfonyl, $C_{1-4}$ alkylaminosulfonyl, di($C_{1-4}$ alkyl)aminosulfonyl, $C_{1-4}$ alkoxycarbonyl, $C_{1-4}$ alkanoyloxy, $C_{1-6}$ alkanoyl, formyl $C_{1-4}$ alkyl, hydroxyimino $C_{1-6}$ alkyl, $C_{1-4}$ alkoxyimino $C_{1-6}$ alkyl or $C_{1-6}$ alkylcarbamoylamino; or

$R^{1E}$ is a group of formula -$SO_2N(R^{cE})R^{c1E}$ [in that case, $R^{cE}$ is hydrogen or $C_{1-4}$ alkyl and $R^{c1E}$ is a hydrogen atom or $C_{1-4}$ alkyl]; or r1 is a group of the following formula ($E^A$), ($E^B$) or ($E^C$):

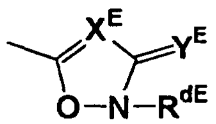
($E^A$)

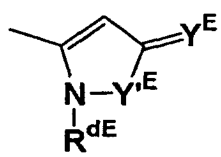
($E^B$)

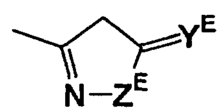
($E^C$)

and, in the above formulae, $X^E$ is CH or a nitrogen atom, $Y^E$ is an oxygen atom or a sulfur atom, $Y'^E$ is an oxygen atom or $NR^{dE}$ and $Z^E$ is $CH_2$, $NR^{dE}$ or an oxygen atom and, in that case, there is one or less ring oxygen and there are at least two ring hetero atoms and, in the above formulae, $R^{dE}$ is a hydrogen atom or $C_{1-4}$ alkyl;

$R^{2E}$ is hydrogen or optionally hydroxy-, cyano- or trifluoromethyl-substituted $C_{1-6}$ alkyl, $C_{2-6}$ alkenyl (in that case, a double bond is not at 1-position), $C_{2-6}$ alkynyl (in that case, a triple bond is not at 1-position), phenyl $C_{1-3}$ alkyl or pyridyl $C_{1-3}$ alkyl;

$R^{3E}$ is a hydrogen atom, methyl or ethyl; and

$R^{4E}$ is an optionally substituted following:

$C_{1-6}$ alkyl, $C_{3-7}$ cycloalkyl $C_{1-3}$ alkyl or $C_{3-7}$ cycloalkyl), or an N-oxide of -$NR^{2E}$- in case chemically possible, or an S-oxide of a sulfur-containing ring in case chemically possible or a pharmaceutically acceptable salt thereof or a hydrolyzable ester or amide in the living body (in that case however, 2-[2-methoxybenzylamino] pyridine-5-carboxylic acid, 4-[2-methoxybenzylamino]benzoic acid, 5-[2,3-dimethoxy- benzylamino]-2-chloro-3-aminosulfonylbenzoic acid and 5-[2,5-dimethoxybenzylamino]-2-hydroxybenzoic acid are to be excluded).

**8.** A compound represented by formula (F) mentioned in the specification of WO 99/47497

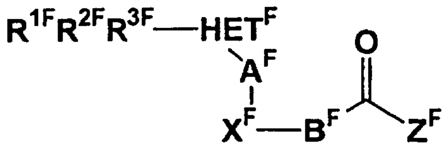

(F)

(in the formula, $HET^F$ is a five- to twelve-membered monocyclic or a bicyclic aromatic ring containing 0 to 3 hetero atom(s) selected from O, $S(O)_{nF}$ and $N(O)_{mF}$ in which $m^F$ is 0 or 1 and $n^F$ is 0, 1 or 2,

$A^F$ is one or two atomic moiety(ies) and is $-W^F-$, -C(O)-, $-C(R^{7F})-W^F-$, $-W^F-C(R^{7F})_2-$, $-CR^{7F}(OR^{20F})-$, $-C(R^{7F})_2-$, $-C(R^{7F})_2-C(OR^{20F})R^{7F}-$, $-C(R^{7F})_2-C(R^{7F})_2-$ or $-CR^{7F}=CR^{7F}-$ in which $W^F$ is O, $S(O)_{nF}$ or $NR^{17}F$

$X^F$ is five- to ten-membered monocyclic, bicyclic or five- to 10-membered monocyclic or bicyclic heteroaryl having 1 to 3 hetero atom(s) selected from O, $S(O)_{nF}$ and $N(O)_{mF}$ which may be substituted with $R^{14F}$ and $R^{15F}$ where $A^f$ and $B^F$ bind to ortho position of aryl or heteroaryl,

$B^F$ is $-(C(R^{18F})_2)_{pF}-Y^F-(C(R^{18F})_2)_{qF}$ where $p^F$ and $q^F$ each independently is 0 to 3,

$Y^F$ is O, $S(O)_{nF}$, $NR^{17F}$ a single bond or $-CR^{18F}=CR^{18F}-$ and, when $Y^F$ is O, $S(O)_{nF}$, $NR^{17F}$ or $-CR^{18F}=CR^{18F}-$, $p^F + q^F$ is 0 to 6 while, when $Y^F$ is a single bond, $p^F + q^F$ is 1 to 6,

$Z^F$ is OH or $NHSO_{2R}{}^{19F}$,

$R^{1F}$, $R^{2F}$ and $R^{3F}$ each independently is H, a halogen atom, lower alkyl, lower alkenyl, lower alkynyl, lower alkenyl-$HET^F(R^{aF})_{4-9}$, $-(C(CR^{4F})_2)_{pF})SR^{5F}$, $-(C(R^{4F})_2)_{pF})OR^{8F}$, $-(C(R^{4F})_2)_{pF}N(R^{6F})_2$, CN, $NO_2$, $-(C(R^{4F})_2)_{pF}C(R^{7F})_3$, $-COOR^{9F}$, $-CON(R^{6F})_2$ or $-(C(R^{4F})_2)_{pF}SS(O)_{nF}R^{10F}$,

each $R^{4F}$ is H, F, $CF_3$ or lower alkyl, or

two $R^{4F}$ are taken in conjunction and represent a at most sixmembered ring which may have one heteroatom selected from O, $S(O)_{nF}$ and $N(O)_{mF}$,

each $R^{5F}$ independently is lower alkyl, lower alkenyl, lower alkynyl, $CF_3$, lower alkyl-$HET^F$, lower alkenyl-$HET^F$or $-(C(R^{18F})_2)_{pF}Ph(R^{11F})_{0-2}$,

each $R^{6F}$ independently is H, lower alkyl, lower alkenyl, lower alkynyl, $CF_3$, phenyl or benzyl, or two $R^{6F}$ binding to N are taken in conjunction and represent a at most six membered ring which may have additional heteroatom selected from O, $S(O)_{nF}$ and $N(O)_{mF}$,

each $R^{7F}$ independently is H, F, $CF_3$ or lower alkyl, or two $F^{7F}$ are taken in conjunction and represent three- to six-membered aromatic or an aliphatic ring containing 0 to 2 heteroatom(s) selected from O, $S(O)_{nF}$ and $N(O)_{mF}$,

each $R^{8F}$ is H or $R^{5F}$,

each $R^{9F}$ independently is H, lower alkyl, lower alkenyl, lower alkynyl, phenyl or benzyl,

each $R^{10F}$ independently is lower alkyl, lower alkenyl, lower alkynyl, $CF_3$, $Ph(R^{11F})_{0-3}$, $CH_2Ph(R^{11F})_{0-3}$ or $N(R^{6F})_2$,

each $R^{11F}$ independently is lower alkyl, $SR^{20F}$, $OR^{20F}$, $N(R^{6F})_2$, $-COOR^{12F}$, $-CON(R^{6F})_2$, $-COR^{12F}$, CN, $CF_3$, $NO_2$ or a halogen atom,

each $R^{12F}$ independently is H, lower alkyl or benzyl,

each $R^{13F}$ independently is H, a halogen atom, lower alkyl, O-lower alkenyl, S-lower alkyl, $N(R^{6F})_2$, $COOR^{12F}$, CN, $CF_3$ or $NO_2$

$R^{14F}$ and $R^{15F}$ each independently is lower alkyl, a halogen atom, $CF_3$, $OR^{16F}$, $S(O)_{nF}R^{16F}$ or $C(R^{16F})_2\ OR^{17F}$,

each $R^{16F}$ independently is H, lower alky, lower alkenyl, phenyl, benzyl or $CF_3$,

each $R^{17F}$ independently is H, lower alkyl or benzyl,

each $R^{18F}$ independently is H, F or lower alkyl, or two $R^{18F}$ are taken in conjunction and represent a three- to six-membered ring which may contain one hetero atom selected from O, $S(O)_{nF}$ and N,

each $R^{19F}$ independently is lower alkyl, lower alkenyl, lower alkynyl, $CF_3$, $HET(R^{aF})_{4-9}$, lower alkyl-$HET(R^{aF})_{4-9}$ or lower alkenyl-$HET(R^{aF})_{4-9}$,

each $R^{20F}$ independently is H, lower alkyl, lower alkenyl, lower alkynyl, $CF_3$ or $Ph(R^{13F})_2$,

each $R^{aF}$ independently is a group selected from the followings:

H, OH, a halogen atom, CN, $NO_2$, amino, $C_{1-6}$ alkyl, $C_{2-6}$ alkenyl, $C_{2-6}$ alkynyl, $C_{1-6}$ alkoxy, $C_{2-6}$ alkenyloxy, $C_{2-6}$ alkynyloxy, $C_{1-6}$ alkylamino, di-($C_{1-6}$ alkyl)amino, $CF_3$, C(O)-$C_{1-6}$ alkyl, C(O)-$C_{2-6}$ alkenyl, C(O)-$C_{2-6}$ alkynyl, COOH, COO-$C_{1-6}$ alkyl, COO-$C_{2-6}$ alkenyl and COO-$C_{2-6}$ alkynyl;

in the group, alkyl, alkenyl, alkynyl and alkyl in alkylamino and dialkylamino may be substituted with one to three of the following group(s):

OH, a halogen atom, aryl, $C_{1-6}$ alkoxy, $C_{2-6}$ alkenyloxy, $C_{2-6}$ alkynyloxy, $CF_3$, CO-$C_{1-6}$ alkyl, CO-$C_{2-6}$ alkenyl, CO-$C_{2-6}$ alkynyl, COOH, COO-$C_{1-6}$ alkyl, COO-$C_{2-6}$ alkenyl, COO-$C_{2-6}$ alkynyl, $NH_2$, NH-$C_{1-6}$ alkyl and N-$C_{1-6}$ $alkyl_2$, or a non-toxic salt thereof.

**9.** The agent for the treatment and/or the prevention of pollakiuria according to claim 1, wherein it is a compound represented by formula (G) mentioned in the specification of WO 00/20371

$$\mathbf{Ar^{1G}\text{-}w^{G}\text{-}Ar^{2G}\text{-}X^{G}\text{-}W^{G}} \qquad \mathbf{(G)}$$

(in the formula, $Ar^{1G}$ is aryl or heteroaryl and may be substituted with $R^{1G}$ or $R^{3G}$,

$R^{1G}$ is $Y^{G}_{MG}$-$R^{2G}$, $Y^{G}_{MG-}AR^{3G}$, a halogen atom, $N(R^{5G})_2$, CN, $NO_2$, $C(R^{6G})_3$, $CON(R^{5G})_2$, $S(O)_{nG}R^{7G}$ or OH,

$Y^G$ is a connecting chain between $Ar^{1G}$ and $R^{2G}$ or $Ar^{3G}$ and contains 0 to 4 carbon atom(s) and at most one hetero atom selected from O, N and S and the connecting chain may contain CO, $S(O)_{nG}$, -C=C- or acetylene or may be further substituted with $R^{2G}$,

$m^G$ is 0 or 1,

$n^G$ is 0, 1 or 2,

$R^{2G}$ is H, F, $CHF_2$, $CF_3$, lower alkyl or hydroxy-$C_{1-6}$ alkyl, or two $R^{2G}$ may be joined together and represent a at most six membered carbon ring which may contain at most one hetero atom selected from O, N and S,

$Ar^{3G}$ is aryl or heteroaryl which may be substituted with $R^{3G}$,

$R^{3G}$ is $R^{4G}$, a halogen atom, halo-$C_{1-6}$ alkyl, $N(R^{5G})_2$, CN, $NO_2$, $C(R^{6G})_3$, $CON(R^{5G})_2$, $OR^{4G}$, $SR^{4G}$ or $S(O)_{nG}R^{7G}$.

$R^{4G}$ is H, lower alkyl, lower alkenyl, lower alkynyl, $CHF_2$ or $CF_3$,

$R^{5G}$ is $R^{4G}$, phenyl or benzyl, or two $R^{5G}$ in combination with a at most six membered ring containing carbon atoms and at most two hetero atom(s) selected from O, N and S,

$R^{6G}$ is H, F, $CF_3$ or lower alkyl, or two $R^{6G}$ may be taken together and represent a at most six membered ring containing carbon atoms and 0 to 2 hetero atom(s) selected from O, N and S,

$R^{7G}$ is lower alkyl, lower alkenyl, lower alkynyl, $CHF_2$, $CF_3$, $N(R^{5G})_2$, $Ph(R^{8G})_2$ or $CH_{2Ph}(R^{8G})_2$,

$R^{8G}$ is $R^{4G}$, $OR^{4G}$, $SR^{4G}$ or a halogen atom

$W^G$ is a three- to six-membered connecting chain containing 0 to 2 hetero atom(s) selected from O, N and S and the connecting chain may contain CO, $S(O)_{mG}$, C=C and acetylene and may be further substituted with $R^{9G}$,

$R^{9G}$ is $R^{2G}$, lower alkenyl, lower alkynyl, $OR^{4G}$ or $SR^{4G}$,

$Ar^{2G}$ is aryl or heteroaryl which may be substituted with $R^{3G}$,

$R^{10G}$ is $R^{4G}$, a halogen atom, $N(R^{5G})_2$, CN, $NO_2$, $C(R^{6G})_3$, $OR^{4G}$, $SR^{4G}$ or $S(O)_{nG}R^{7G}$,

$X^G$ is a connecting group which is substituted at the ortho position to $Ar^{2G}$ based on $W^G$ and it contains 0 to 4 carbon atom(s) and at most one hetero atom selected from O, N and S, may contain CO, $S(O)_{nG}$, C=C or acetylene, and may be further substituted with $R^{11G}$,

$R^{11G}$ has the same meaning as $R^{9G}$,

$Q^G$ is a group selected from COOH, tetrazole, $SO_3H$, hydroxamic acid, $CONHSO_2R^{12G}$ and $SO_2NHCOR^{12G}$,

$R^{12G}$ is a group selected from $CF_3$, lower alkyl, lower alkenyl, lower alkynyl and $Z^9Ar^{4G}$,

$Z^G$ is 0 to 4 connecting chain(s) which may be substituted with $R^{13G}$,

$R^{13}G$ has the same meaning as $R^{9G}$,

$Ar^{4G}$ is aryl or heteroaryl which may be substituted with $R^{14G}$,

$R^{14G}$ is $R^{10G}$ or NHCOMe.), or a non-toxic salt thereof.

**10.** The agent for the treatment and/or the prevention of pollakiuria according to claim 1, wherein it is

(1 ) 6-[(2S,3S)-3-(4-chloro-2-methylphenylsulfonyl-aminomethyl)-bicyclo[2.2.2]-octan-2-yl]-5Z-hexenoic acid,
(2) 8-chlorodibenz[b,f][1.4]oxazepine-10(11H)-carboxylic acid 2-[1-oxo-3-(4-pyridinyl)propyl]hydrazide monohydrochloride or
(3) N-(3,5-dimethylisoxazol-4-ylsulfonyl)-6-[N-(5-chloro-2-(isobutyloxy)benzyl)-N-ethylamino]pyridazine-3-carboxamide.

## INTERNATIONAL SEARCH REPORT

International application No.
PCT/JP02/12000

**A. CLASSIFICATION OF SUBJECT MATTER**
Int.Cl[7] A61K45/00, 31/19, 31/501, 31/553, A61P13/00, 13/10, 43/00

According to International Patent Classification (IPC) or to both national classification and IPC

**B. FIELDS SEARCHED**

Minimum documentation searched (classification system followed by classification symbols)
Int.Cl[7] A61K45/00, 31/19, 31/501, 31/553, A61P13/00, 13/10, 43/00

Documentation searched other than minimum documentation to the extent that such documents are included in the fields searched

| | | | |
|---|---|---|---|
| Jitsuyo Shinan Koho | 1926-1992 | Toroku Jitsuyo Shinan Koho | 1994-1996 |
| Kokai Jitsuyo Shinan Koho | 1971-1992 | Jitsuyo Shinan Toroku Koho | 1996-2002 |

Electronic data base consulted during the international search (name of data base and, where practicable, search terms used)
CA(STN), MEDLINE(STN), BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | EP 878465 A2 (Ono Pharmaceutical Co., Ltd.),<br>18 November, 1998 (18.11.98),<br>Page 2, lines 20 to 23; page 25, lines 7 to 13<br>& JP 11-29548 A & JP 2000-103778 A | 1,2,4,10<br>3,5-9 |
| X<br>Y | EP 947500 A1 (Ono Pharmaceutical Co., Ltd.),<br>06 October, 1999 (06.10.99),<br>Page 31, lines 13 to 22; page 32, lines 29 to 37<br>& WO 98/27053 A1 | 1-3<br>4-10 |
| X<br>Y | JP 2000-7646 A (Ono Pharmaceutical Co., Ltd.),<br>11 January, 2000 (11.01.00),<br>Par. Nos. [0005], [0191]<br>(Family: none) | 1<br>2-10 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 February, 2003 (17.02.03) | Date of mailing of the international search report<br>04 March, 2003 (04.03.03) |
| Name and mailing address of the ISA/<br>Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/12000

C (Continuation). DOCUMENTS CONSIDERED TO BE RELEVANT

| Category* | Citation of document, with indication, where appropriate, of the relevant passages | Relevant to claim No. |
|---|---|---|
| Y | WO 97/00863 A1 (Zeneca Ltd.), 09 January, 1997 (09.01.97), Full text & JP 11-508878 A & EP 835246 A1 | 7,10 |
| Y | WO 92/19617 A2 (Searle, G.D., and Co.), 12 November, 1992 (12.11.92), Full text & JP 6-507408 A | 5,10 |
| Y | WO 96/06822 A1 (Zeneca Ltd.), 07 March, 1996 (07.03.96), Full text & JP 10-504836 A | 6 |
| Y | WO 99/47497 A1 (Merck Frosst Canada and Co.), 23 September, 1999 (23.09.99), Full text & JP 2002-506851 A | 8 |
| Y | WO 00/20371 A1 (Merck Frosst Canada and Co.), 13 April, 2000 (13.04.00), Full text & JP 2002-526517 A | 9 |

Form PCT/ISA/210 (continuation of second sheet) (July 1998)

INTERNATIONAL SEARCH REPORT

International application No.
PCT/JP02/12000

Although it is stated at the end of claim 6 "- - - a hydrolyzable ester or amide.", it has been checked with the agent of the applicant that this expression is a mistake for "- - - a remedy and/or preventive for urinary frequency as set forth in claim 1 which is a hydrolyzable ester or amide.".

Although it is stated at the end of claim 8 "a compound represented by - - - - or its salt.", it has been also checked with the agent of the applicant that this expression is a mistake for "- - - a remedy and/or preventive for urinary frequency as set forth in claim 1 which is a compound represented by - - - - or its salt."

Form PCT/ISA/210 (extra sheet) (July 1998)